# EUROPÄISCHE PATENTSCHRIFT

(11) **EP 0 438 206 B1**
(45) Veröffentlichungstag und Bekanntmachung des Hinweises auf die Patenterteilung: **21.08.1996**
(21) Anmeldenummer: 91250008.9
(22) Anmeldetag: 16.01.1991
(51) Int. Cl.: C07D 471/08, A61K 49/00, A61K 51/06, A61K 31/495, A61K 31/555

(54) **6-Ring enthaltende makrocyclische Tetraaza-Verbindungen, Verfahren zu ihrer Herstellung und diese enthaltende pharmazeutische Mittel**
6-Ring-containing macrocyclic tetraaza compounds, processes for their preparation, and pharmaceutical agents containing them
Composés tétraaza-macrocycliques contenant un noyau à six membres, procédés pour leur préparation et agents pharmaceutiques les contenant

(30) Priorität: 18.01.1990 DE 4001655
(43) Veröffentlichungstag der Anmeldung: 24.07.1991
(73) Patentinhaber: SCHERING AKTIENGESELLSCHAFT, D-13342 Berlin (DE)
(72) Erfinder: Gries, Heinz, Dr., W-1000 Berlin 31 (DE); Radüchel, Bernd, Dr., W-1000 Berlin 28 (DE); Platzek, Johannes, Dr., W-1000 Berlin 33 (DE); Weinmann, Hanns-Joachim, Dr., W-1000 Berlin 38 (DE); Press, Wolf-Rüdiger, W-1000 Berlin 42 (DE)

(56) Entgegenhaltungen:
- EP-A- 0 232 751
- EP-A- 0 255 471
- EP-A- 0 299 795
- EP-A- 0 352 218
- EP-A- 0 391 766
- TETRAHEDRON, vol. 37, 1981, Seiten 767 - 772; H. STETTER et al.: "Darstellungund Komplexbildung von Polyazacycloalkan-N-essigsäuren"
- CHEMICAL ABSTRACTS, vol. 113, no. 7, 13 August 1990 Columbus, Ohio, USA H.TAKALO et al.: "Preparation of new macrocyclic polyamines containing the 4-[phenylethynyl]pyridine subunit" Seite 701; ref. no. 59139G & J. Heterocycl. Chem. 1990, 27[2], 167-9

## Beschreibung

Die Erfindung betrifft den in den Patentansprüchen gekennzeichneten Gegenstand, d.h. 6-Ring enthaltende makrocyclische Tetraaza-Komplexbildner, -Komplexe und -Komplexsalze, diese Verbindungen enthaltende Mittel, ihre Verwendung als Diagnostika und Therapeutika sowie Verfahren zur Herstellung dieser Verbindungen und Mittel.

Metallkomplexe sind schon zu Beginn der 50er Jahre als Kontrastmittel für die Radiologie in Betracht gezogen worden. Die damals eingesetzten Verbindungen waren aber derart toxisch, daß eine Verwendung beim Menschen nicht in Betracht kam. Es war daher durchaus überraschend, daß sich bestimmte Komplexsalze als ausreichend verträglich erwiesen haben, so daß eine routinemäßige Anwendung am Menschen für diagnostische Zwecke in Erwägung gezogen werden konnte. Als erster Vertreter dieser Substanzklasse ist das in der europäischen Patentanmeldung mit der Publikationsnummer 71564 beschriebene Dimegluminsalz des Gd DTPA (Gadolinium-III-Komplex der Diethylentriaminpentaessigsäure) unter dem Namen Magnevist^{(R)} als Kontrastmittel für die Kernspintomographie registriert worden. Der Schwerpunkt der Anwendung liegt bei Erkrankungen des Zentralnervensystems.

Ein wesentlicher Grund für die gute Anwendbarkeit von Gd DTPA in der Klinik liegt in der hohen Wirksamkeit bei der Kernspintomographie, insbesondere bei vielen Hirntumoren. Wegen seiner guten Wirksamkeit kann Gd DTPA mit 0,1 mmol/kg Körpergewicht sehr viel niedriger dosiert werden als beispielsweise Röntgenkontrastmittel in vielen Röntgenuntersuchungen.

Als weiterer Vertreter der Komplexsalze hat sich das in der deutschen Patentanmeldung 34 01 052 beschriebene Megluminsalz des Gd DOTA (Gadolinium-III-Komplex der 1,4,7,10-Tetraazacyclododecan-tetraessigsäure) für diagnostische Zwecke bewährt.

Nun besteht aber der Wunsch, Chelate auch höher dosiert einzusetzen. Das ist besonders zum Nachweis bestimmter Erkrankungen außerhalb des Zentralnervensystems mit Hilfe der Kernspintomographie (NMR-Diagnostik), ganz besonders aber bei der Verwendung von Chelaten als Röntgenkontrastmittel, der Fall.

Um dabei die Volumenbelastung des Körpers möglichst gering zu halten, ist es notwendig, hochkonzentrierte Chelatlösungen zu verwenden. Die bisher bekannten Chelate sind vor allem wegen ihrer zu hohen Osmolalität dafür wenig geeignet.

Es besteht daher ein Bedarf an Chelaten, die eine geringere Osmolalität als die vorbekannten Chelate aufweisen. Gleichzeitig müssen jedoch die Voraussetzungen für die Anwendung dieser Verbindungen am Menschen bezüglich des Abstands zwischen der wirksamen und der im Tierversuch toxischen Dosis (die therapeutische Breite), der Organspezifität, der Stabilität, der kontrastverstärkenden Wirkung, der Verträglichkeit sowie der Löslichkeit der Komplexverbindungen erfüllt sein.

Der Erfindung liegt somit die Aufgabe zugrunde, diese Verbindungen und Mittel zur Verfügung zu stellen, sowie ein möglichst einfaches Verfahren zu ihrer Herstellung zu schaffen. Diese Aufgabe wird durch die vorliegende Erfindung erfüllt.

Die erfindungsgemäßen Komplexverbindungen und die aus ihnen bereiteten Lösungen erfüllen die genannten Anforderungen in überraschender Weise. Sie besitzen eine verringerte Osmolalität sowie eine günstigere therapeutische Breite und/oder Stabilität und Lagerfähigkeit der chemischen Bestandteile der Lösung und/oder Organspezifität und/oder kontrastverstärkende Wirkung (z.B. Relaxivität) und/oder Verträglichkeit (z.B. geringere kardiovaskuläre oder allergieartige Nebenwirkungen) als die bisher gebräuchlichen Diagnostika.

Auch ohne spezifische Maßnahmen erlaubt ihre Pharmakokinetik die Verbesserung der Diagnose zahlreicher Erkrankungen. Die Komplexe werden zum größten Teil unverändert und rasch wieder ausgeschieden, so daß insbesondere auch im Falle der Verwendung relativ toxischer Metallionen trotz hoher Dosierung keine schädlichen Wirkungen beobachtet werden.

Die praktische Anwendung der neuen Komplexe und Komplexbildner wird auch durch deren günstige chemische Stabilität erleichtert.

Ein weiterer wesentlicher Vorteil der beschriebenen Komplexe und Komplexbildner ist deren außerordentliche chemische Vielseitigkeit. Neben dem Zentralatom lassen sich die Eigenschaften durch die Wahl vielfältiger Substituenten im Makrocyclus und/oder der Salzbildner den Anforderungen an Wirksamkeit, Pharmakokinetik, Verträglichkeit, Löslichkeit, Handhabbarkeit usw. anpassen. So kann z.B. eine in der Diagnostik und Therapie sehr erwünschte Spezifität der Verbindungen für Strukturen im Organismus, für bestimmte biochemische Substanzen, für Stoffwechselvorgänge, für Zustände der Gewebe oder Körperflüssigkeiten, erzielt werden.

Die erfindungsgemäßen makrocyclischen Verbindungen werden durch die allgemeine Formel I gekennzeichnet: 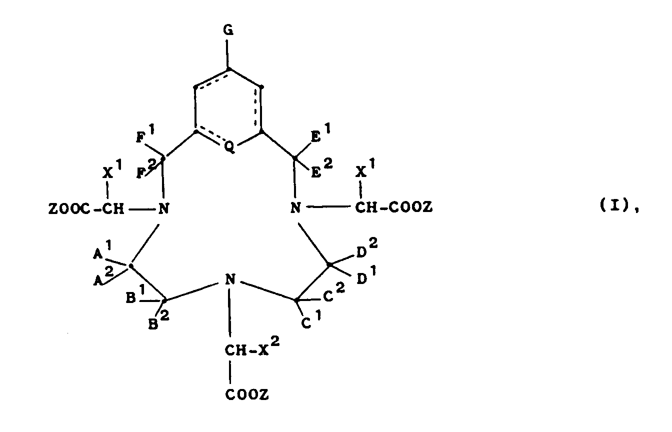 worin
- ...: für eine Einfach- oder Doppelbindung,
- Q: für ein Stickstoffatom oder den Rest NH,
- X¹: für ein Wasserstoffatom, eine -(CH₂)ₙ-R¹- oder 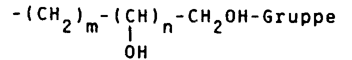 mit
- n: in der Bedeutung der Ziffern 1 bis 5,
- m: in der Bedeutung der Ziffern 0 bis 2 und
- R¹: in der Bedeutung eines Wasserstoffatoms oder einer Hydroxygruppe,
- X²: für X¹ oder eine -(CH₂)ₙ-(O)ₗ-(CH₂)ₖ-(C₆H₄)_{q}-R²-Gruppe mit
- k: in der Bedeutung der Ziffern 0 bis 4,
- l und q: in der Bedeutung der Ziffern 0 oder 1 und
- R²: in der Bedeutung eines Wasserstoffatoms, einer C₁-C₄-Alkoxygruppe, einer funktionellen Gruppe oder gebunden über diese funktionelle Gruppe eines Bio- oder Makromoleküls,
- A¹, A², B¹, B², C¹, C², D¹, D², E¹, E², F¹ und F²: unabhängig voneinander jeweils für X²
- G: für R² oder einen über K gebundenen zweiten Makrocyclus der allgemeinen Formel II 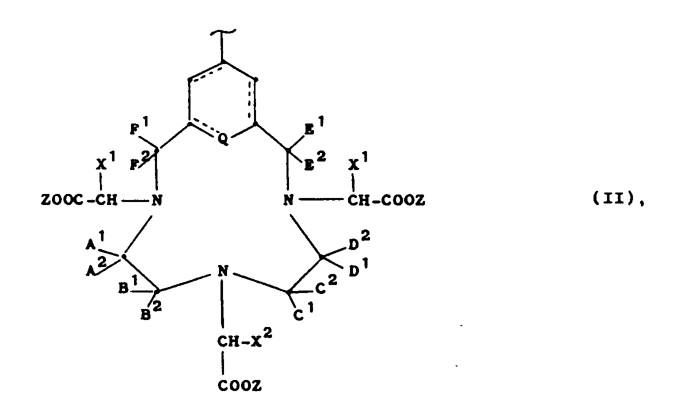 mit K in der Bedeutung einer direkten Bindung, einer Bis(carbonylamino)-gruppe (-NH-CO-CO-NH-) oder einer C₁-C₁₄-Alkylengruppe, die gegebenenfalls an den Enden Carbonyl-(>CO) oder Carbonylamino (-NH-CO-)-gruppen oder Sauerstoffatome trägt und gegebenenfalls ein oder mehrere Sauerstoffatom(e), Hydroxymethylen- (-CH-OH-) gruppe(n) CH(X²)COOZ-, acyl- oder hydroxyacylsubstituierte Iminogruppen oder ein bis zwei C-C-Doppel- und/oder C-C-Dreifachbindungen enthält,
- Z: für ein Wasserstoffatom und/oder ein Metallionenäquivalent eines Elements der Ordnungszahlen 21-29, 31, 32, 37-39, 42-44, 49 oder 57-83
stehen mit der Maßgabe, daß die 12 Ringsubstituenten A¹ bis F² für mindestens 8 Wasserstoffatome stehen, daß X¹ und X² nur dann gleichzeitig für Wasserstoffatome stehen, wenn mindestens einer der Ringsubstituenten A¹ bis F² nicht für ein Wasserstoffatom, A¹ bis D² nicht für (CH₂)₁₋₆-H und E¹ bis F² nicht für (CH₂)₁₋₅-H steht und daß der Makrocyclus der allgemeinen Formel I nicht mehr als ein Bio- oder Makromolekül enthält und daß gewünschtenfalls der Rest der CO₂H-Gruppen als Ester oder Amid vorliegt, sowie deren Salze mit anorganischen und/oder organischen Basen, Aminosäuren oder Aminosäureamiden.

Bevorzugt sind Tetraaza-Verbindungen der allgemeinen Formel III 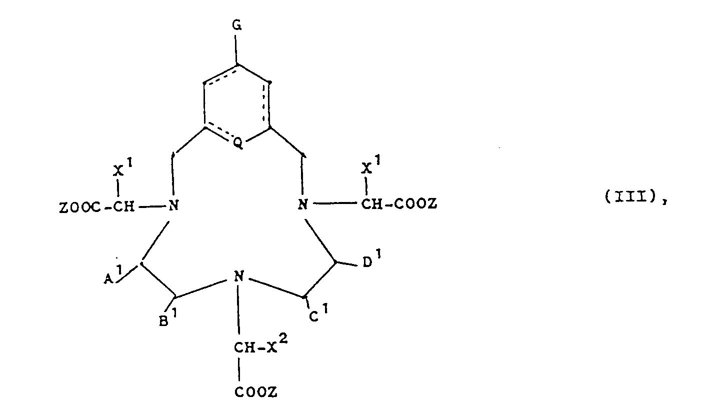 worin
- ...: für eine Einfach- oder Doppelbindung,
- Q: für ein Stickstoffatom oder den Rest NH,
- X¹: für ein Wasserstoffatom, eine -(CH₂)ₙ-R¹- oder 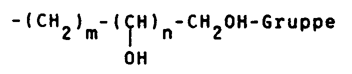 mit
- n: in der Bedeutung der Ziffern 1 bis 5,
- m: in der Bedeutung der Ziffern 0 bis 2 und
- R¹: in der Bedeutung eines Wasserstoffatoms oder einer Hydroxygruppe,
- X²: für X¹ oder eine -(CH₂)ₙ-(O)ₗ-(CH₂)ₖ-(C₆H₄)_{q}-R²-Gruppe mit
- k: in der Bedeutung der Ziffern 0 bis 4,
- l und q: in der Bedeutung der Ziffern 0 oder 1 und
- R²: in der Bedeutung eines Wasserstoffatoms, einer C₁-C₄-Alkoxygruppe, einer funktionellen Gruppe oder gebunden über diese funktionelle Gruppe eines Bio- oder Makromoleküls,
- A¹, B¹, C¹ und D¹: unabhängig voneinander jeweils für X²
- G: für R² oder einen über K gebundenen zweiten Makrocyclus der allgemeinen Formel IV 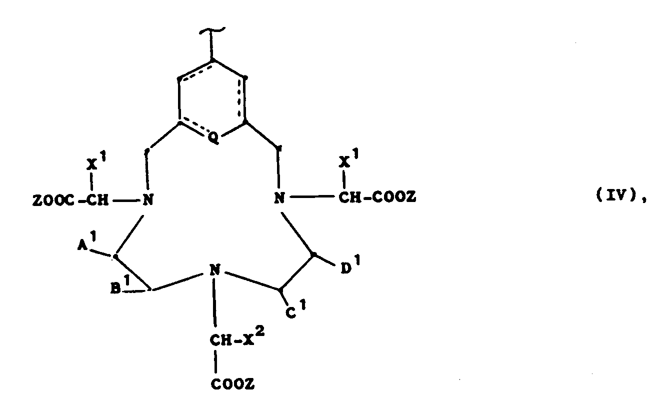 mit K in der Bedeutung einer direkten Bindung, einer Bis(carbonylamino)-gruppe (-NH-CO-CO-NH-) oder einer C₁-C₁₄-Alkylengruppe, die gegebenenfalls an den Enden Carbonyl-(>CO) oder Carbonylamino (-NH-CO-)-gruppen oder Sauerstoffatome trägt und gegebenenfalls ein oder mehrere Sauerstoffatom(e), Hydroxymethylen- (-CH-OH-) gruppe(n) CH(X²)COOZ-, acyl- oder hydroxyacylsubstituierte Iminogruppen oder ein bis zwei C-C-Doppel- und/oder C-C-Dreifachbindungen enthält,
- Z: für ein Wasserstoffatom und/oder ein Metallionenäquivalent eines Elements der Ordnungszahlen 21-29, 31, 32, 37-39, 42-44, 49 oder 57-83
stehen mit der Maßgabe, daß X¹ und X² nur dann gleichzeitig für Wasserstoffatome stehen, wenn mindestens einer der 4 Ringsubstituenten A¹, B¹, C¹ und D¹ nicht für ein Wasserstoffatom oder für (CH₂)₁₋₆-H steht, und daß gewünschtenfalls der Rest der CO₂H-Gruppen als Ester oder Amid vorliegt, sowie deren Salze mit anorganischen und/oder organischen Basen, Aminosäuren oder Aminosäureamiden.

Verbindungen der allgemeinen Formel I mit Z in der Bedeutung von Wasserstoff werden als Komplexbildner und mit mindestens zwei der Substituenten Z in der Bedeutung eines Metallionenäquivalents als Metallkomplexe bezeichnet.

Das Element der oben genannten Ordnungszahl, welches das Zentralion des physiologisch verträglichen Komplexsalzes bildet, kann für den angestrebten Verwendungszweck des erfindungsgemäßen diagnostischen Mittels selbstverständlich auch radioaktiv sein.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Für die Verwendung der erfindungsgemäßen Mittel in der Nuklearmedizin muß das Zentralion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium und Iridium.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Als bevorzugte Gruppen für X¹ seien CH₂OH, CH₂CH₂OH und CHOHCH₂OH, als bevorzugte Gruppen für X², A¹, B¹, C¹ und/oder D¹, CH₂OH, CH₂CH₂OH, CH₂OCH₂C₆H₅, CHOHCH₂OH, CH₂C₆H₄OCH₃, CH₂C₆H₅, CH₂C₆H₄O(CH₂)₃COOH, CH₂C₆H₄NCS genannt, wobei die übrigen Ringsubstituenten A², B², C², D², E¹, E², F¹ und F² bevorzugt für Wasserstoff stehen.

Die für K stehende Alkylenkette, an die der zweite Makrocyclus II bzw. IV gebunden ist, trägt an den Enden gegebenenfalls Carbonyl-(CO), Carbonylamino-(NH-CO)-gruppen oder Sauerstoffatome und enthält 1 - 14 Kohlenstoffatome. Sie kann durch ein oder mehrere Sauerstoffatom(e), Hydroxymethylen(-CHOH-), gruppe(n) CH(X²)COOZ-, acyl- oder hydroxyacyl-substituierte Iminogruppen oder ein bis zwei C-C-Doppel- und/oder C-C-Dreifachbindungen unterbrochen sein. Die beiden Makrocyclen können aber auch durch eine direkte Bindung verknüpft sein. Als gegebenenfalls hydroxylierte Acylgruppen kommen dabei Acyl-Reste mit bis zu 10 Kohlenstoffatomen infrage. Beispielhaft genannt seien der Acetyl-, Propionyl-, Butyryl-, Benzoyl- und Hydroxyacetyl-Rest.

Die Alkylenkette kann gerad- oder verzweigtkettig, gesättigt oder ungesättigt und gegebenenfalls wie beschrieben unterbrochen sein. Sie kann bis zu 4 Sauerstoffatome und/oder bis zu 3 Carboxymethyliminogruppen enthalten.

Beispiele für die alkylenkette sind:
-(CH₂)₂-, -CH₂-O-CH₂-, -(CH₂)₄-, -CH₂-CH₂-O-CH₂-CH₂-, -(CH₂-O-CH₂)₂-, -(CH₂-O-CH₂)₃-, -CH₂-CH₂-(O-CH₂-CH₂)₃-, CH₂CH₂-(O-CH₂-CH₂)₄-, 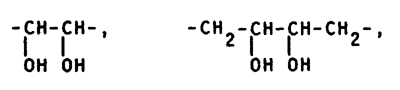 -C≡C-C≡C-, 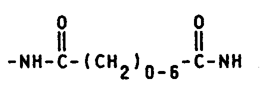 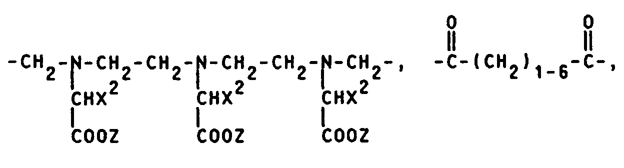 -O-(CH₂)₁₋₆-O-, 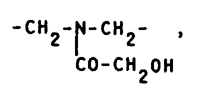 -(CH₂)₂CH=CH-(CH₂)₂- .

Bevorzugt sind die beiden Makrocyclen durch eine direkte Bindung oder die Gruppe 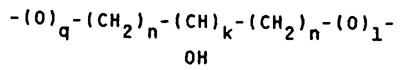 verknüpft.

Bevorzugte funktionelle Gruppen, für die R² stehen kann, sind beispielsweise die Maleimidobenzoyl-, 3-Sulfomaleimidobenzoyl-, 4-(Maleimidomethyl]-cyclohexylcarbonyl-, 4-[3-Sulfo-(maleimidomethyl)]cyclohexyl-carbonyl-, 4-(p-Maleimidophenyl)-butyryl-, 3-(2-Pyridyldithio)propionyl-, Methacryl-oyl-(pentamethylen)amido-, Bromacetyl-, Jodacetyl-, 3-Jodpropyl-, 2-Bromethyl-, 3-Mercaptopropyl-, 2-Mercaptoethyl-, Phenylenisothiocyanat-, 3-Aminopropyl-, Benzylester-, Ethylester-, t-Butylester-, Amino-, Hydroxy-, C₁-C₆-Alkylamino-, Aminocarbonyl-, Hydrazino-, Hydrazinocarbonyl-, Maleimido-, Methacrylamido-, Methacryloylhydrazinocarbonyl-, Maleimidamidocarbonyl-, Halogeno-, Mercapto-, Hydrazinotrimethylenhydrazinocarbonyl-, Aminodimethylenamidocarbonyl-, Bromcarbonyl-, Phenylendiazonium-, Isothiocyanat-, Semicarbazid-, Thiosemicarbazid-, Isocyanat-Gruppe.

Zur Verdeutlichung seien einige ausgewählte Gruppen aufgeführt: 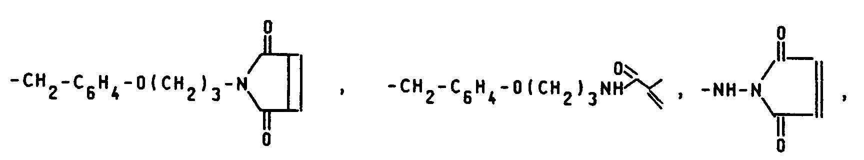 -CH₂-C₆H₄-0(CH₂)₅CO₂CH₂C₆H₅, -CH₂-C₆H₄-O-CH₂-CO₂CH₂C₆H₅, -CH₂-C₆H₄-0(CH₂)₅CONHNH₂, 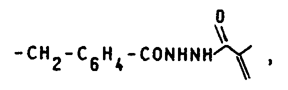 -CH₂-C₆H₄-0(CH₂)₄-SH, -CH₂-C₆H₄-0(CH₂)₃NHNH₂, 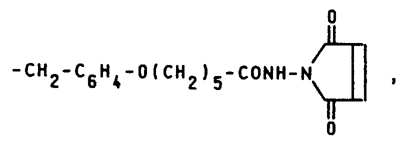 -CH₂-C₆H₄-O(CH₂)₃Br, C0₂-C₆H₄-NO₂, -CH₂-C₆H₄-0(CH₂)₅CONHNH-(CH₂)₃-NHNH₂, -CH₂-NHNH₂, -CH₂-SH, -CH₂CONHNH₂, -(CH₂)₃SH, CH₂-C₆H₄-0-CH₂COBr, -C₆H₄NHCOCH₂Br, 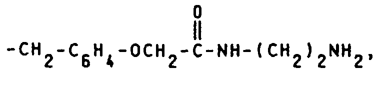 -CH₂-C₆H₄-NH₂, -C₆H₄-N₂, -C₆H₄NCS, 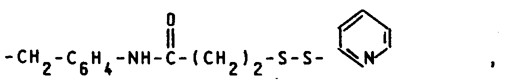 -NHCO-NH-NH₂, -NHCS-NH-NH₂, 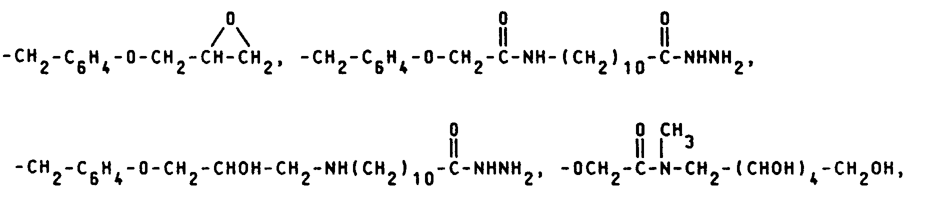 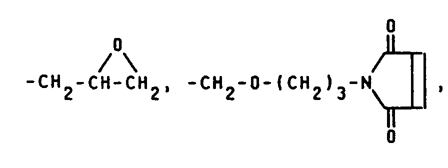 -CH₂-0-(CH₂)₄-SH, -CH₂-0-(CH₂)₃-NHNH₂, 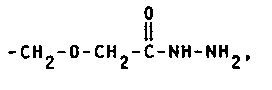 -CH₂-0-CH₂-CH₂-NH₂, 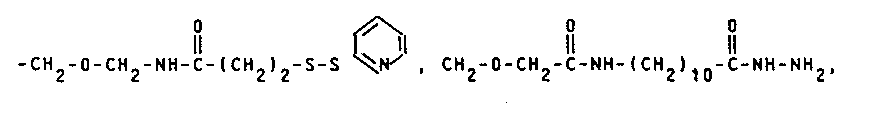 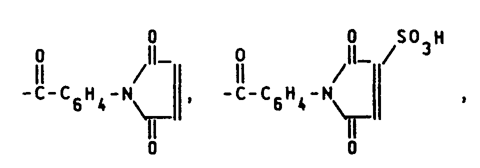 -C≡C-C≡C-R, -C≡C-CH=CRR*',* 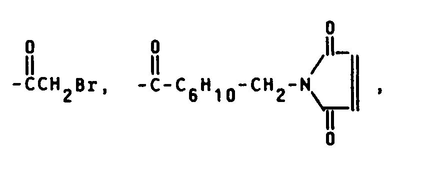 -NH-CO-CH₂-Br, -NH-CO-CH₂Cl, 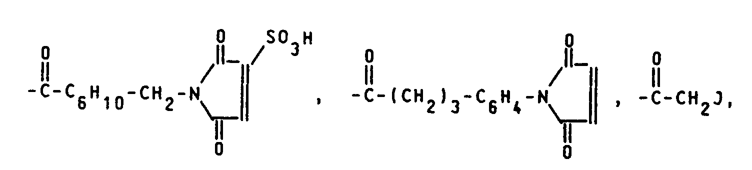 -(CH₂)₃SH, -(CH₂)₃NH₂, -C₆H₄SCN, 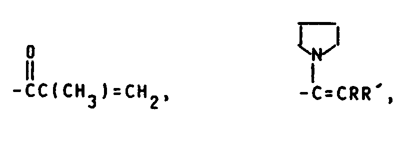 C₆H₄CH₂Br, 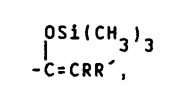 -CH₂Br, -CH₂J, -CH=CH-CH₂Br, -OSO₂C₆H₄CH₃, -SO₂Cl, -SOCl, 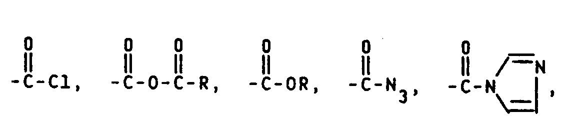 -CH=CH-CO₂R,
wobei R und R' gleich oder verschieden sind und jeweils für ein Wasserstoffatom, einen gesättigten oder ungesättigten gegebenenfalls durch eine Phenylgruppe substituierten C₁-C₂₀-Alkylrest oder eine Phenylgruppe stehen. Besonders bevorzugt sind die -NCS, -NO₂, -OH, -NHNH₂, NHCOCH₂Br, -NHCOCH₂Cl, -CO₂H und -CON₃-Gruppe.

Die restlichen aciden Wasserstoffatome, das heißt diejenigen, die nicht durch das Zentralion substituiert worden sind, können gegebenenfalls ganz oder teilweise durch Kationen anorganischer und/oder organischer Basen oder Aminosäuren ersetzt sein. Die entsprechenden Säuregruppen können auch ganz oder teilweise in Ester oder Amide überführt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Geeignete Ester sind vorzugsweise diejenigen mit einem C₁-C₆-Alkylrest; genannt seien beispielsweise der Methyl-, Ethyl- und tertiär-Butyl-, Benzyl- und 4-Methoxybenzylrest.

Sollen die Carbonsäuregruppen zumindest teilweise als Amide vorliegen, so kommen als Reste gesättigte, ungesättigte, gerad- oder verzweigtkettige oder cyclische Kohlenwasserstoffe mit bis zu 5 C-Atomen infrage, die gegebenenfalls durch 1 bis 3 Hydroxy- oder C₁-C₄-Alkoxy-Gruppen substituiert sind. Beispielsweise genannt seien die Methyl-, Ethyl-, 2-Hydroxyethyl-, 2-Hydroxy-1-(hydroxymethyl)-ethyl-, 1-(Hydroxymethyl)-ethyl-, Propyl-, Isopropenyl-, 2-Hydroxypropyl-, 3-Hydroxypropyl-, 2,3-Dihydroxypropyl-, Butyl-, Isobutyl-, Isobutenyl-, 2-Hydroxybutyl-, 3-Hydroxybutyl-, 4-Hydroxybutyl-, 2-, 3- und 4-Hydroxy-2-methylbutyl-, 2- und 3-Hydroxyisobutyl-, 2,3,4-Trihydroxybutyl-, 1,2,4-Trihydroxybutyl-, Pentyl-, Cyclopentyl- und 2-Methoxyethylgruppe. Der Amidrest kann auch ein unter Einschluß des Amid-Stickstoffs gebildeter heterocyclischer 5- oder 6-Ring sein. Beispielhaft genannt seien: der Pyrrolidinyl-, Piperidyl-, Pyrazolidinyl-, Pyrrolinyl-, Pyrazolinyl-, Piperazinyl-, Morpholinyl-, Imidazolidinyl-, Oxazolidinyl-, Thiazolidinyl-Ring.

Die erfindungsgemäßen Verbindungen weisen die eingangs geschilderten gewünschten Eigenschaften auf. Sie enthalten die für ihre Verwendung benötigten Metallionen im Komplex stabil gebunden.

Der für Nebenwirkungen wie Schmerzen, Schädigungen der Blutgefäße und Herz-Kreislauf-Störungen verantwortliche Wert der Osmolalität ist im Vergleich zu Magnevist deutlich verringert (Beispiel 1b: 0,55 [osmol/kg] gegenüber Magnevist 1,96 [osmol/kg], 0,5 mol/l bei 37 °C).

Überraschend groß ist der Wert für die ein MaB der Bildgebung beim MRI darstellende Größe der Relaxivity; die Signalverstärkung im Plasma ließ sich z.B. im Falle der Verbindung des Beispiels 1b gegenüber Magnevist um das Doppelte steigern.

Ein weiterer Vorteil der vorliegenden Erfindung liegt darin, daß nunmehr Komplexe mit hydrophilen oder lipophilen Substituenten zugänglich geworden sind. Dadurch ist die Möglichkeit gegeben, Verträglichkeit und Pharmakokinetik dieser Komplexe durch chemische Substitution zu steuern.

Durch die Wahl geeigneter Bio- oder Makromoleküle (s. weiter unten) in R² erhält man erfindungsgemäße Komplexe, die eine überraschend hohe Gewebe- und Organspezifität aufweisen.

Die Herstellung der erfindungsgemäßen Verbindungen erfolgt dadurch, daß man in Verbindungen der allgemeinen Formel I' 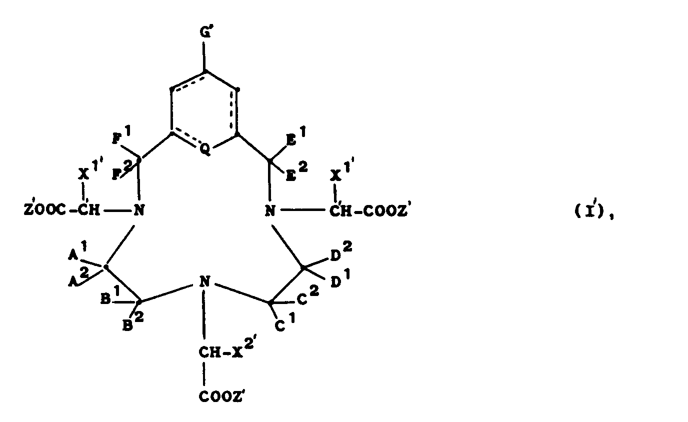 worin
- G': X^{1'} und X^{2'} jeweils für G, X¹ und X², bei denen die darin enthaltenen Hydroxy- und funktionellen Gruppen in geschützter Form bzw. als Vorstufe vorliegen, und
- Z': für ein Wasserstoffatom oder eine Säureschutzgruppe
stehen,
die Schutzgruppen abspaltet, gegebenenfalls die gewünschte funktionelle Gruppe generiert, gewünschtenfalls die so erhaltenen Komplexbildner der allgemeinen Formel I mit Z in der Bedeutung von Wasserstoff in an sich bekannter Weise mit mindestens einem Metalloxid oder Metallsalz eines Elements der Ordnungszahlen 21-29, 31, 32, 37-39, 42-44, 49 oder 57-83 umsetzt, gewünschtenfalls die funktionellen Gruppen an ein Bio- oder Makromolekül bindet - wobei die Komplexierung vor oder nach der Abspaltung der Schutzgruppen für die Hydroxy- und funktionellen Gruppen bzw. Generierung der funktionellen Gruppen und Bindung an ein Makro- oder Biomolekül erfolgen kann - und anschließend, falls gewünscht, noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester oder Amide überführt.

Als Säureschutzgruppen Z' kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Z' kann auch für ein Alkalimetall stehen.

Die Abspaltung der Schutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 bis 50 °C, saure Verseifung mit Mineralsäuren oder im Fall von z.B. tert.-Butylestern mit Hilfe von Trifluoressigsäure.

Als Hydroxyschutzgruppen kommen z.B. die Benzyl-, 4-Methoxybenzyl-, 4-Nitrobenzyl-, Trityl-, Diphenylmethyl-, Trimethylsilyl-, Dimethyl-t-butylsilyl-, Diphenyl-t-butylsilylgruppe infrage.

Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Falle von Polyolen können die Hydroxygruppen auch in Form von Ketalen mit z.B. Aceton, Acetaldehyd, Cyclohexanon oder Benzaldehyd geschützt sein.

Die in X^{1'} und X^{2'} vorliegenden Hydroxygruppen können auch durch intramolekulare Veresterung mit den α-ständigen Carboxylgruppen zu den entsprechenden Lactonen geschützt vorliegen.

Die Hydroxyschutzgruppen können nach den dem Fachmann bekannten Literaturmethoden, z.B. durch Hydrogenolyse, reduktive Spaltung mit Lithium/Ammoniak, Säurebehandlung der Ether und Ketale oder Alkalibehandlung der Ester freigesetzt werden (siehe z.B. "Protective Groups in Organic Synthesis", T.W. Greene, John Wiley and Sons 1981).

Die Synthese von dimeren Verbindungen, d.h. von Verbindungen, die einen zweiten Makrocyclus der allgemeinen Formel II bzw. IV enthalten, erfolgt nach literaturbekannten Verfahren, zum Beispiel über eine Additions/Eliminierungs-Reaktion eines Amins mit einer Carbonylverbindung (z.B. Säurechlorid, gemischtes Anhydrid, aktivierter Ester, Aldehyd); zweier aminsubstituierter Ringe mit einer Dicarbonylverbindung (z.B. Oxalylchlorid, Glutardialdehyd); zweier p-nitrosubstituierter Nitroxide mit Bisalkoholaten [vgl. E. Klingsberg, The Chemistry of Heterocyclic Compounds, Interscience Publishers New York, p. 514, (1961)]; zweier Ringe, die je eine nukleophile Gruppe aufweisen, mit einer zwei Fluchtgruppen tragenden Alkylenverbindung oder im Falle terminaler Acetylene durch oxidative Kupplung (Cadiot, Chodkiewicz in Viehe "Acetylenes", 597-647, Marcel Dekker, New York, 1969).

Die die Ringe verknüpfende Kette kann anschließend durch Folgereaktionen modifiziert werden (z.B. Hydrierung).

Die Synthese von direkt verknüpften Verbindungen (d.h. K steht für eine direkte Bindung, siehe Beispiel 4) kann durch Cyclisierung von Tetrahalogenomethyl-4,4'-bispyridinen erfolgen (s. weiter unten).

Die Synthese der Edukte I' erfolgt durch Alkylierung von Verbindungen der allgemeinen Formel V 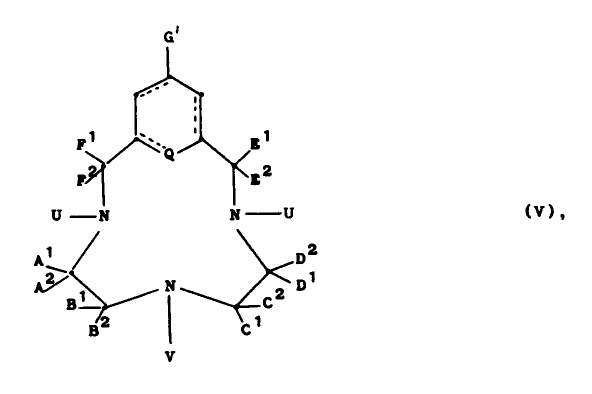 worin
- U: für Wasserstoff und V für eine Aminoschutzgruppe bzw.
- U: für Aminoschutzgruppen und V für Wasserstoff
stehen,
wobei U und V auch identisch sein können,
mit Verbindungen der allgemeinen Formel VI 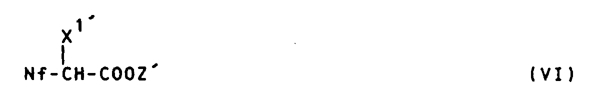 bzw. Verbindungen der allgemeinen Formel VII 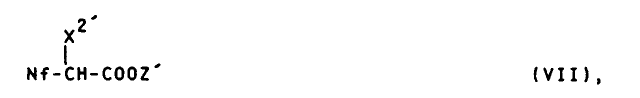 worin
Nf für ein Nucleofug wie z.B. Cl, Br, J, CH₃-C₆H₄SO₃, CH₃SO₃, 4-NO₂-C₆H₄SO₃, CF₃SO₃ steht.

Die in X^{1'} und X^{2'} gegebenenfalls enthaltenen Hydroxygruppen können auch zusammen mit dem OZ'-Rest ein Lacton bilden.

Als Alkylierungsreagenz seien beispielhaft genannt: Bromessigsäure, Chloressigsäure, Bromessigsäuremethylester, Bromessigsäure-t-butylester, Chloressigsäurebenzylester, 2-Chlor-3-benzyloxypropansäure-Natriumsalz (EP 0 325 762), 2-Brom-3-benzyloxypropansäure-t-butylester (J. Gen. Chem., USSR 36, 52, 1966), 3,4-O-Isopropyliden-2-p-tolylsulfonyl-3,4-dihydroxybuttersäureethylester (Synth. Comm. 19, 3077, 1989), α-Brom-γ-butyrolacton.

Als Aminoschutzgruppen U bzw. V seien beispielhaft genannt: Formyl, Trifluoracetat, Benzoat, 4-Nitrobenzoat, Acetat, Tosylat, Mesylat, Benzyl, 4-Nitrobenzyl, 4-Methoxybenzyl, Trimethylsilyl, Dimethyl-t-butylsilyl.

Die Alkylierung der Verbindungen der allgemeinen Formel V zu den Edukten der allgemeinen Formel I' mit den Verbindungen der allgemeinen Formel VI bzw. VII erfolgt in polaren aprotischen Lösungsmitteln wie z.B. Dimethylformamid, Acetonitril, Dimethylsulfoxid, wäßriges Tetrahydrofuran, Dioxan oder Hexamethylphosphorsäuretriamid in Gegenwart eines Säurefängers, wie z.B. tertiäres Amin (z.B. Triäthylamin, Trimethylamin, N,N-Dimethylaminopyridin, 1,5-Diazabicyclo[4.3.0]nonen-5 (DBN), 1,5-Diazabicyclo[5.4.0]undecen-5 (DBU), Alkali-, Erdalkalicarbonat, -hydrogencarbonat oder -hydroxid (z.B. Natrium-, Lithium-, Magnesium-, Calcium-, Barium-, Kalium-carbonat, -hydroxid und -hydrogencarbonat) bei Temperaturen zwischen -10 °C und 120 °C, vorzugsweise zwischen 0 °C und 50 °C, wobei gegebenenfalls katalytische Mengen Jodid oder Bromid zugesetzt werden können.

Nach Abspaltung der restlichen Aminoschutzgruppe(n) nach den dem Fachmann bekannten Methoden (z.B. saure oder basische Hydrolyse, Hydrogenolyse, reduktive Abspaltung mit Alkalimetallen in flüssigem Ammoniak, Reaktion mit Tetrabutylammoniumfluorid) wird/werden in einer zweiten Alkylierungsreaktion mit VII bzw. VI die verbleibende(n) Aminofunktion(en) umgesetzt, so daß Verbindungen mit X¹ ≠ X² erhalten werden.

Die Umwandlung einer Vorstufe des im Endprodukt enthaltenen gewünschten 6-gliedrigen Ringes erfolgt nach den dem Fachmann bekannten Methoden. Beispielhaft erwähnt seien die Hydrierung von Pyridin [Advan. Catal. 14, 203 (1963)], Deoxygenierung von Nitroxidringen [E. Klingsberg, The Chemistry of Heterocyclic Compounds, Volume 14, part 2, Interscience Publishers New York, p. 120 (1961)], Umwandlungen und Einführung von funktionellen Gruppen am 6-Ring, z.B. Freisetzung von phenolischen Hydroxygruppen [J. Org. Chem. 53, 5 (1988)], Einführung von Halogensubstituenten [E. Klingsberg, The Chemistry of Heterocyclic Compounds, Volume 14, Part 2, Interscience Publishers New York, p. 341 (1961), Houben-Weyl, Methoden der organischen Chemie, Band V/3, 651 (1962)].

Die Funktionalisierung von 4-Halogenpyridinderivaten (z.B. Azidaustausch) im Phasentransfer-Verfahren unter Verwendung von 18-Krone-6 oder Tetrabutylammoniumhalogenid als Katalysator ist in "Phase Transfer Reactions" (Fluka Compendium Vol. 2, Walter E. Keller, Georg Thieme Verlag Stuttgart, New York) beschrieben. Nach dem Fachmann bekannten Methoden (z.B. katalytische Hydrierung, Houben-Weyl "Methoden der organischen Chemie", Band 11/1, S. 539) oder Umsetzung mit Raney-Nickel/Hydrazin (Deutsche Patentanmeldung 3 150 917) kann eine so erhaltene Azidgruppe in eine Aminofunktion überführt werden. Diese kann mit literaturbekannten Methoden (z.B. mit Thiophosgen in einem Zweiphasensystem, S. Scharma, Synthesis 1978, 803, D. K. Johnson, J. Med. Chem. 1989, Vol. 32, 236) in eine Isothiocyanatgruppe umgewandelt werden.

Durch Umsetzung einer Aminofunktion mit einem Halogenessigsäurehalogenid kann eine α-Halogenacetamidgruppe generiert werden (JACS 1969, Vol. 90, 4508; Chem. Pharm. Bull. 29 (1), 128, 1981), die ebenso wie z.B. die Isothiocyanatgruppe zur Kopplung an Bio- und Makromoleküle geeignet ist.

Die Synthese der Verbindungen der allgemeinen Formel V wird durch Cyclisierung nach literaturbekannten Methoden [zum Beispiel Org. Synth. 58, 86 (1978), Makrocyclic Polyether Syntheses, Springer Verlag Berlin, Heidelberg, New York (1982), Coord. Chem. Rev. 3, 3 (1968), Ann. Chem. 1976, 916, J. Org. Chem. 49, 110 (1984)] durchgeführt; einer der beiden Reaktanten trägt am Kettenende zwei Fluchtgruppen, der andere zwei Stickstoffatome, die nukleophil diese Fluchtgruppen verdrängen.

Als Beispiel seien genannt die Umsetzung von A¹-D²-substituierten Diethylentriaminen, deren endständige Stickstoffatome nucleophil die Fluchtgruppen von z.B. 2,6-Dihalogenmethyl-pyridinen, 2,6-Ditosylmethyl-pyridinen oder 2,6-Dimesylmethyl-pyridinen verdrängen. Zur Synthese von direkt verknüpften Dimeren werden in die Cyclisierungsreaktion 2,2' ,6,6'-Tetrachlormethyl-4,4'-Bispyridine (siehe z.B. Synthesis 552, 1989) eingesetzt.

Die Stickstoffatome sind gegebenenfalls geschützt (zum Beispiel als Tosylate oder Trifluoracetate) und werden vor der nachfolgenden Alkylierungsreaktion nach literaturbekannten Verfahren freigesetzt (die Tosylate z.B. mit Mineralsäuren, Alkalimetallen in flüssigem Ammoniak, Bromwasserstoffsäure und Phenol, RedAl^{(R)}, Lithiumaluminiumhydrid, Natrium-Amalgam, vgl. z.B. Liebigs Ann. Chem. 1977, 1344, Tetrahedron Letters 1976, 3477; die Trifluoracetate z.B. mit Mineralsäuren oder Ammoniak in Methanol, vgl. z.B. Tetrahedron Letters 1967, 289).

Zur Herstellung von an den Stickstoffatomen unterschiedlich substituierten Makrocyclen können diese Atome in den Edukten mit unterschiedlichen Schutzgruppen, z.B. mit Tosylat- und Benzylgruppen, versehen werden. Letztere werden dann ebenfalls nach literaturbekannten Methoden (bevorzugt durch Hydrierung, z.B. EP-Patentanmeldung 232 751) entfernt.

Werden Diester in die Cyclisierungsreaktion eingesetzt, so müssen die so erhaltenen Diketoverbindungen nach dem Fachmann bekannten Verfahren, zum Beispiel mit Diboran, reduziert werden.

Es können auch entsprechend substituierte endständige Bisaldehyde bzw. Bisketone, z.B. 2,6-Bisacetyl-pyridine, mit den jeweils gewünschten endständigen Bisaminen cyclisiert werden; die Reduktion der so erhaltenen Schiffschen Basen erfolgt nach literaturbekannten Methoden, z.B. durch katalytische Hydrierung [Helv. Chim. Acta 61, 1376 (1978)].

Die Herstellung der als Ausgangssubstanzen für die Cyclisierung benötigten Amine erfolgt analog literaturbekannten Methoden (z.B. EP 299 795).

Ausgehend von einer N-geschützten Aminosäure erhält man durch Umsetzung mit einem partiell geschützten Diamin (zum Beispiel nach der Carbodiimidmethode), Abspaltung der Schutzgruppen und Diboranreduktion ein Triamin.

Als Substituenten, der in die für eine Bindung an ein Makro- oder Biomolekül geeignete funktionelle Gruppe überführt werden kann, sind unter anderem Hydroxy- und Nitrobenzyl-, Hydroxy- und Carboxylalkyl- sowie Thioalkylreste mit bis zu 20 Kohlenstoffatomen geeignet. Sie werden nach dem Fachmann bekannten Literaturverfahren [Chem. Pharm. Bull. 33, 674 (1985), Compendium of Org. Synthesis Vol. 1-5, Wiley and Sons, Inc., Houben-Weyl, Methoden der organischen Chemie, Band VIII, Georg Thieme Verlag, Stuttgart, J. Biochem. 92, 1413, (1982)] in die gewünschten Substituenten (zum Beispiel mit der Amino-, Hydrazino-, Hydrazinocarbonyl-, Epoxid-, Anhydrid-, Methacryloylhydrazinocarbonyl-, Maleimidamidocarbonyl-, Halogeno-, Halogenocarbonyl-, Mercapto-, Isothiocyanatgruppe als funktioneller Gruppe) umgewandelt, wobei im Falle des Nitrobenzylrestes zunächst eine katalytische Hydrierung (zum Beispiel nach P.N. Rylander, Catalytic Hydrogenation over Platinum Metals, Academic Press 1967) zum Aminobenzylderivat vorgenommen werden muß.

Beispiele für die Umwandlung von an aromatische oder aliphatische Reste gebundenen Hydroxy- oder Aminogruppen sind die in geeigneten Lösungsmitteln wie Tetrahydrofuran, Dimethoxyethan oder Dimethylsulfoxid, zweiphasigen wäßrigen Systemen, wie z.B. Wasser/Dichlormethan, in Gegenwart eines Säurefängers wie zum Beispiel Natriumhydroxid, Natriumhydrid oder Alkali oder Erdalkalicarbonaten wie zum Beispiel Natrium-, Magnesium-, Kalium-, Calciumcarbonat oder Poly-(4-vinylpyridin) Reillex^{(R)} bei Temperaturen zwischen 0 °C und dem Siedepunkt des jeweiligen Lösungsmittels, vorzugsweise jedoch zwischen 20 °C und 60 °C, durchgeführten Umsetzungen mit einem Substrat der allgemeinen Formel VIII

Nf-L-Fu (VIII),

worin L für einen aliphatischen, aromatischen, arylaliphatischen, verzweigten, geradkettigen oder cyclischen Kohlenwasserstoffrest mit bis zu 20 Kohlenstoffatomen und Fu für die gewünschte endständige funktionelle Gruppe, gegebenenfalls in geschützter Form. stehen (DE-OS 34 17 413).

Als Beispiele für Verbindungen der allgemeinen Formel VIII seien genannt
Br(CH₂)₂NH₂, Br(CH₂)₃OH, BrCH₂COOCH₃, BrCH₂CO₂^{t}Bu, ClCH₂CONHNH₂, Br(CH₂)₄CO₂C₂H₅, BrCH₂COBr, BrCH₂CONH₂, ClCH₂COOC₂H₅, BrCH₂CONHNH₂, 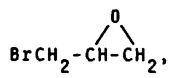 CF₃SO₃(CH₂)₃Br, BrCH₂C≡CH, BrCH₂CH=CH₂, BrCH₂C₆H₄NCS.

Umwandlungen von Carboxy-Gruppen können zum Beispiel nach der Carbodiimid-Methode (Fieser, Reagents for Organic Syntheses 10, 142), über ein gemischtes Anhydrid [Org. Prep. Proc. Int. 7, 215 (1975)] oder über einen aktivierten Ester (Adv. Org. Chem. Part B, 472) durchgeführt werden.

Die so erhaltenen komplexbildenden Liganden (sowie die Komplexe) können auch an Bio- oder Makromoleküle geknüpft sein, von denen bekannt ist, daß sie sich in dem zu untersuchenden Organ oder Organteil besonders anreichern. Solche Moleküle sind beispielsweise Enzyme, Hormone, Polysaccharide wie Dextrane oder Stärken, Porphyrine, Bleomycine, Insulin, Prostaglandine, Steroidhormone, Aminozucker, Aminosäuren, Peptide wie Polylysin, Proteine (wie zum Beispiel Immunoglobuline, monoklonale Antikörper, Lektine), Lipide (auch in Form von Liposomen) und Nukleotide vom DNA- oder RNA-Typ. Besonders hervorzuheben sind Konjugate mit Albuminen, wie Humanserumalbumin, Antikörpern, wie zum Beispiel monoklonale, für tumorassoziierte Antigene spezifische Antikörper oder Antimyosin. Anstelle von biologischen Makromolekülen können auch geeignete synthetische Polymere wie Polyethylenimine, Polyamide, Polyharnstoffe, Polyether wie Polyethylenglykole und Polythioharnstoffe angeknüpft werden. Die hieraus gebildeten pharmazeutischen Mittel eignen sich beispielsweise zur Anwendung in der Tumor- und Infarkt-Diagnostik sowie Tumortherapie. Monoklonale Antikörper (zum Beispiel Nature 256, 495, 1975) haben gegenüber polyklonalen Antikörpern die Vorzüge, daß sie spezifisch für eine antigene Determinante sind, eine definierte Bindungsaffinität besitzen, homogen sind (damit wird ihre Reindarstellung wesentlich einfacher) und in Zellkulturen in großen Mengen herstellbar sind. Als solche sind zum Beispiel für die Tumordarstellung monoklonale Antikörper bzw. deren Fragmente Fab und F(ab')₂ geeignet, die zum Beispiel spezifisch sind für humane Tumore des Gastrointestinaltraktes, der Brust, der Leber, der Blase, der Keimdrüsen und von Melanomen [Cancer Treatment Repts. 68, 317, (1984), Bio Sci 34, 150, (1984)] oder gegen Carcinomembryonales Antigen (CEA), Humanes Choriogonadotropin (β-HCG) oder andere tumorständige Antigene, wie Glycoproteine, gerichtet sind [New Engl. J. Med. 298, 1384, (1973), US-P 4,331,647]. Geeignet sind unter anderem auch Anti-Myosin, Anti-Insulin- und Anti-Fibrin-Antikörper (US-P 4,036,945).

Coloncarcinome lassen sich mit Hilfe von mit Gadolinium(III)-Ionen komplexierten Konjugaten mit dem Antikörper 17-1A (Centocor, USA) NMR-diagnostisch nachweisen.

Für Leberuntersuchungen beziehungsweise für die Tumordiagnostik eignen sich beispielsweise Konjugate oder Einschlußverbindungen mit Liposomen, die beispielsweise als unilamellare oder multilamellare Phosphatidylcholin-Cholesterol-Vesikel eingesetzt werden.

Im Falle der Antikörper-Konjugate darf die Bindung des Antikörpers an den Komplex oder Liganden nicht zum Verlust oder zur Verminderung der Bindungsaffinität und Bindungsspezifität des Antikörpers zum Antigen führen. Dies kann entweder durch Bindung an den Kohlenhydrat-Anteil im Fc-Teil des Glycoproteins bzw. in den Fab oder F(ab')₂-Fragmenten oder durch Bindung an Schwefelatome des Antikörpers bzw. der Antikörper-Fragmente erfolgen.

Im ersten Fall muß zunächst eine oxidative Spaltung von Zuckereinheiten zur Generation kopplungsfähiger Formylgruppen durchgeführt werden. Diese Oxidation kann auf chemischem Wege mit Oxidationsmitteln wie z.B. Perjodsäure, Natriummetaperjodat oder Kaliummetaperjodat nach literaturbekannten Methoden (zum Beispiel J. Histochem and Cytochem. 22, 1084, 1974) in wäßriger Lösung in Konzentrationen von 1 bis 100, vorzugsweise 1 bis 20 mg/ml, und einer Konzentration des Oxidationsmittels zwischen 0,001 bis 10 mMol, vorzugsweise 1 bis 10 mMol, in einem pH-Bereich von ca. 4 bis 8 bei einer Temperatur zwischen 0 bis 37 °C und einer Reaktionsdauer zwischen 15 Minuten und 24 Stunden vorgenommen werden. Auch auf enzymatischem Wege kann die Oxidation, beispielsweise mit Hilfe von Galaktoseoxidase, in einer Enzymkonzentration von 10 - 100 Einheiten/ml, einer Substratkonzentration von 1 bis 20 mg/ml, bei einem pH-Wert von 5 bis 8, einer Reaktionsdauer von 1 bis 8 Stunden und einer Temperatur zwischen 20 und 40 °C, durchgeführt werden (zum Beispiel J. Biol. Chem. 234, 445, 1959).

An die durch Oxidation generierten Aldehyde werden Komplexe oder Liganden mit geeigneten funktionellen Gruppen, wie zum Beispiel Hydrazin, Hydrazid, Hydroxylamin, Phenylhydrazin, Semicarbazid und Thiosemicarbazid, durch Reaktion zwischen 0 - 37 °C, bei einer Reaktionsdauer von 1 bis 65 Stunden, einem pH-Wert zwischen ca. 5,5 und 8, einer Antikörperkonzentration von 0,5 bis 20 mg/ml und einem molaren Verhältnis des Komplexbildners zum Antikörperaldehyden von 1:1 bis 1000:1 gebunden. Die anschließende Stabilisierung des Konjugats erfolgt durch Reduktion der Doppelbindung, z.B. mit Natriumborhydrid oder Natriumcyanoborhydrid; das Reduktionsmittel wird dabei in einem 10 bis 100fachen Überschuß verwendet (zum Beispiel J. Biol. Chem. 254, 4359, 1979).

Die zweite Möglichkeit der Bildung von Antikörper-Konjugaten geht aus von einer schonenden Reduktion der Disulfid-Brücken des Immunoglobulin-Moleküls; hierbei werden die empfindlicheren Disulfid-Brücken zwischen den H-Ketten des Antikörper-Moleküls gespalten, während die S-S-Bindungen der Antigenbindenden Region intakt bleiben, so daß praktisch keine Verminderung der Bindungsaffinität und -spezifität des Antikörpers eintritt (Biochem. 18, 2226, 1979, Handbook of Experimental Immunology, Vol. 1, Second Edition, Blackwell Scientific Publications, London 1973, Chapter 10). Diese freien Sulfhydryl-Gruppen der interHKetten Regionen werden dann mit geeigneten funktionellen Gruppen von Komplexbildnern oder Metallkomplexen bei 0 bis 37 °C, einem pH-Wert von ca. 4 bis 7, und einer Reaktionsdauer von 3 bis 72 Stunden unter Ausbildung einer kovalenten Bindung, die die Antigen-Bindungsregion des Antikörpers nicht beeinflußt, umgesetzt. Als geeignete reaktive Gruppen seien beispielsweise genannt: Halogenalkyl-, Halogenacetyl-, p-Mercuribenzoat-, Isothiocyanat-, Thiol-, Epoxidgruppen sowie Gruppen, die einer MichaelAdditions-Reaktion, wie zum Beispiel Maleinimide, Methacrylogruppen (zum Beispiel J. Amer. Chem. Soc. 101, 3097, 1979), zu unterwerfen sind.

Zur Verknüpfung der Antikörperfragmente mit den Komplexen bzw. den Liganden gibt es zusätzlich eine Reihe geeigneter, oft auch kommerziell erhältlicher bifunktioneller "Linker" (siehe zum Beispiel Pierce, Handbook and General Catalogue 1986), die sowohl gegenüber den SH-Gruppen der Fragmente als auch gegenüber den Amino- bzw. Hydrazinogruppen der Komplexe reaktiv sind.

Als Beispiele seien genannt:
m-Maleimidobenzoyl-N-hydroxysuccinimidester (MBS),
m-Maleimidobenzoyl-N-sulfosuccinimidester (Sulfo-MBS),
N-Succinimidyl-[4-(Iodacetyl)-amino]benzoesäureester (SIAB),
Succinimidyl-4(N-maleimidomethyl)-cyclohexan-1-carbonsäureester (SMCC),
Succinimidyl-4(p-maleimidophenyl)-buttersäureester (SMPB),
N-Succinimidyl-3-(2-pyridyldithio)-propionsäureester (SDPD),
4-[3-(2,5-Dioxo-3-pyrrolinyl)-propionyloxy]-3-oxo-2,5-diphenyl-2,3-dihydrothiophen-1,1-dioxid,
Acetylalanylleucylalanylaminobenzyl,
Acetamido-p-thioureidobenzyl.

Es können auch Bindungen nicht-kovalenter Art zur Kopplung genutzt werden, wobei sowohl ionische als auch van der Waals- und Wasserstoffbrücken-Bindungen in wechselnden Anteilen und Stärke (Schlüssel-Schloß-Prinzip) zur Bindung beitragen können (zum Beispiel Avidin-Biotin, Antikörper-Antigen). Auch Einschlußverbindungen (host-guest) kleinerer Komplexe in größere Cavitäten beim Makromolekül sind möglich.

Das Kopplungsprinzip besteht darin, zunächst ein bifunktionelles Makromolekül herzustellen, indem man entweder ein gegen ein Tumorantigen gerichtetes Antikörper-Hybridom mit einem gegen einen erfindungsgemäßen Komplex gerichtetes zweiten Antikörper-Hybridom fusioniert oder die beiden Antikörper chemisch über einen Linker (beispielsweise in der im J. Amer. Chem. Soc. 101, 3097 (1979) angegebenen Weise) miteinander verknüpft oder den gegen das Tumorantigen gerichteten Antikörper, gegebenenfalls über einen Linker, an Avidin (bzw. Biotin) bindet [D.J. Hnatowich et al., J. Nucl. Med. 28, 1294 (1987)]. Anstelle der Antikörper können auch ihre entsprechenden F(ab)- bzw. F(ab')₂-Fragmente verwendet werden. Für die pharmazeutische Anwendung injiziert man zunächst das bifunktionelle Makromolekül, das sich am Zielort anreichert, und dann im zeitlichen Abstand die erfindungsgemäße Komplexverbindung [gegebenenfalls an Biotin (bzw. Avidin) gebunden], die in-vivo am Zielort angekoppelt wird und dort ihre diagnostische oder therapeutische Wirkung entfalten kann. Darüberhinaus können sich auch andere Kopplungsmethoden wie beispielsweise das in Protein Tailoring Food Med. Uses [Am. Chem. Soc. Symp.] (1985), 349, beschriebene "Reversible Radiolabeling" zur Anwendung kommen.

Mit der sogenannten Festphasen-Kopplung steht eine besonders einfache Methode zur Herstellung von Antikörper-Konjugaten bzw. Antikörperfragment-Konjugaten zur Verfügung: Der Antikörper wird an eine stationäre Phase (z.B. einen Ionenaustauscher), der sich zum Beispiel in einer Glassäule befindet. gekoppelt. Durch sukzessives Spülen der Säule mit einer zur Generierung von Aldehyd-Gruppen geeigneten Lösung, Waschen, Spülen mit einer Lösung des funktionalisierten Komplexes und schließlich Eluieren des Konjugats werden sehr hohe Konjugat-Ausbeuten erhalten.

Dieses Verfahren erlaubt die automatische und kontinuierliche Produktion beliebiger Mengen an Konjugaten.

Auch andere Kopplungsschritte können auf diese Art und Weise durchgeführt werden.

So können zum Beispiel durch die Sequenz Papain-Reduktion/bifunktioneller Linker/funktionalisierter Komplex bzw. Ligand Fragment-Konjugate hergestellt werden.

Die so gebildeten Verbindungen werden anschließend vorzugsweise chromatographisch über Ionenaustauscher auf einer Fast-Protein-Liquid-Chromatography-Anlage gereinigt.

Die Herstellung der erfindungsgemäßen Metallkomplexe erfolgt in der Weise, wie sie in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21 - 29, 42, 44, 57 - 83 in Waser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome durch Kationen anorganischer und/oder organischer Basen oder Aminosäuren substituiert.

Die Einführung der gewünschten Metallionen kann dabei sowohl vor als auch nach der Abspaltung der Schutzgruppen für die Hydroxy- und funktionellen Gruppen bzw. vor oder nach der Generierung der funktionellen Gruppen und Bindung an ein Makro- oder Biomolekül erfolgen.

Die Neutralisation eventuell noch vorhandener freier Carboxygruppen erfolgt mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Säuregruppen im Komplex ganz oder teilweise in zum Beispiel Ester oder Amide zu überführen. Dies kann durch nachträgliche Reaktion am fertigen Komplex geschehen (z.B. durch erschöpfende Umsetzung der freien Carboxy-Gruppen mit Dimethylsulfat).

Die Konjugate aus Antikörper und Komplex werden vor der in-vivo-Anwendung nach Inkubation mit einem schwachen Komplexbildner, wie zum Beispiel Natriumcitrat, Natrium-Ethylen-diamintetraessigsäure dialysiert, um schwachgebunde Metallatome zu entfernen.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern (wie zum Beispiel Diethylentriaminpentaessigsäure) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 0,1 µMol - 1 Mol/l des Komplexsalzes und werden in der Regel in Mengen von 0,1 µMol - 5 mMol/kg dosiert. Sie sind zur enteralen und parenteralen Applikation bestimmt. Die erfindungsgemäßen Komplexverbindungen kommen zur Anwendung.
1. für die NMR- und Röntgen-Diagnostik in Form ihrer Komplexe mit den Ionen der Elemente mit den Ordnungszahlen 21 - 29, 42, 44 und 57-83;
2. für die Radiodiagnostik und Radiotherapie in Form ihrer Komplexe mit den Radioisotopen der Elemente mit den Ordnungszahlen 27, 29, 31, 32, 37-39, 43, 49, 62, 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100 bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001 - 5 mMol/kg. vorzugsweise 0,005 - 0,5 mMol/kg, dosiert. Details der Anwendung werden zum Beispiel in H.J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983).

Die erfindungsgemäßen Verbindungen können auch in der Radioimmuno- oder Strahlentherapie verwendet werden. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronenemittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. [Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der Methode, z.B. Brachytherapie.

Die erfindungsgemäßen therapeutischen Mittel werden parenteral, vorzugsweise i.v. appliziert.

Details der Anwendung von Radiotherapeutika werden z.B. in R.W. Kozak et al. TIBTEC, Oktober 1986, 262, diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler - "Einführung in die Röntgendiagnostik", G. Thieme, Stuttgart, New York (1977) diskutiert.

Insgesamt ist es gelungen, neue Komplexbildner, Metallkomplexe und Metallkomplexsalze zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstands.

### Beispiel 1

a) **3,6,9-Tris[dihydro-2(3H)-furanon-3yl]-3,6,9,15-tetraazabicyclo[9.3.1]pentadeca-1(15),11,13-trien**
Zu 10 g (48,48 mmol) 3,6,9,15-Tetraazabicyclo[9.3.1]pentadeca-1(15),11,13-trien, 33,5 g (242,38 mmol) Kaliumcarbonat und 805 mg (4,85 mmol) Kaliumjodid in 200 ml Acetonitril gibt man 40 g (242,38 mmol) α-Bromo-γ-butyrolacton. Diese Mischung wird 48 Stunden unter Rückfluß erhitzt. Man dampft im Vakuum zur Trockene ein. Der Rückstand wird in 500 ml Methylenchlorid aufgenommen und 3 mal mit 150 ml Wasser extrahiert. Die organische Phase wird über Magnesiumsulfat getrocknet und im Vakuum eingedampft. Der Rückstand wird durch Chromatographie an Kieselgel gereinigt. (Laufmittel: Methylenchlorid/Methanol= 15:1).
Ausbeute: 7,11 g (32 % der Theorie) eines leicht gelbgefärbten Öls, das beim Stehenlassen erstarrt.

| Analyse: | | | |
|---|---|---|---|
| C 60,25 | H 6,59 | N 12,22 | (Ber.) |
| C 60,18 | H 6,64 | N 12,17 | (Gef.) |

b) **Gadolinium-Komplex der 3,6,9,15-Tetraazabicyclo[9.3.1]pentadeca-1(15),11,13-trien-3,6,9-tris[α-(2-hydroxyethyl)essigsäure]**
6,7 g (14,61 mmol) der Titelverbindung aus Beispiel 1a werden in 50 ml entionisiertem Wasser gelöst und der pH durch Zugabe von 1 normaler Salzsäure auf 5,5 gebracht. Man fügt 2,65 g (7,3 mmol) Gadoliniumoxid zu und kocht 3 Stunden unter Rückfluß. Die abgekühlte Lösung wird mit je 10 ml saurem Ionenaustauscher (IR 120) und 10 ml basischem Austauscher (IRA 410) eine Stunde gerührt. Man filtriert vom Austauscher ab und kocht das Filtrat für 1 Stunde mit Aktivkohle. Nach Filtration und Gefriertrocknung erhält man 9,25 g (95 % der Theorie) eines amorphen, farblosen Pulvers (enthält 8,3 % Wasser laut Analyse).

| Analyse (auf Wasser korrigiert): | | | | |
|---|---|---|---|---|
| C 41,43 | H 4,99 | N 8,40 | Gd 23,58 | (Ber.) |
| C 41,35 | H 5,09 | N 8,34 | Gd 23,50 | (Gef.) |

c) **Europium-Komplex der 3,6,9,15-Tetraazabicyclo[9.3.1]pentadeca-1(15),11,13-trien-3,6,9-tris[α-(2-hydroxyethyl)essigsäure]**
In analoger Weise erhält man mit ¹⁵¹Eu₂O₃ den entsprechenden Europium-Komplex.

| Analyse (auf Wasser korrigiert): | | | | |
|---|---|---|---|---|
| C 41,76 | H 5,03 | N 8,47 | Eu 22,97 | (Ber.) |
| C 41,68 | H 5,12 | N 8,39 | Eu 22,88 | (Gef.) |

### Beispiel 2

a) **3,6,9,15-Tetraazabicyclo[9.3.1]pentadeca-1(15),11,13-trien-3,6,9-tris[α-(benzyloxymethyl)essigsäure]**
10 g (48,48 mmol) 3,6,9,15-Tetraazabicyclo[9.3.1]pentadeca-1(15),11,13-trien und 114,71 g (484,8 mmol) des Natriumsalzes der 2-Chloro-3-benzyloxypropionsäure in 200 ml Wasser werden 48 Stunden auf 70°C erhitzt. Die Lösung wird mit 400 ml Wasser verdünnt und 300 ml 2N Salzsäure zugegeben. Man extrahiert 5 mal mit je 200 ml Methylenchlorid. Die Wasserphase wird im Vakuum eingedampft. Der Rückstand wird in 300 ml Ethanol gelöst und vom Natriumchlorid abfiltriert. Man dampft im Vakuum ein und chromatographiert das zurückbleibende Öl an Kieselgel (Laufmittel: Ethanol/Wasser=20:1). Die Hauptfraktionen werden im Vakuum eingedampft und in 50 ml 5 %iger Salzsäure gelöst. Die Lösung wird auf eine Säule gefüllt mit Reillex^{R} (=Poly-4-vinylpyridin)gegeben und das Produkt mit einer Mischung aus Wasser/Methanol 3:1 eluiert. Nach Eindampfen der Hauptfraktionen erhält man 12,93 g (36 % der Theorie) eines stark hygroskopischen Feststoffes (9,1 % Wasser laut Analyse).

| Analyse (auf Wasser korrigiert): | | | |
|---|---|---|---|
| C 66,47 | H 6,53 | N 7,56 | (Ber.) |
| C 66,38 | H 6,60 | N 7,48 | (Gef.) |

b) **3,6,9,15-Tetraazabicyclo[9.3.1]pentadeca-1(15),11,13-trien 3,6,9-tris[α-(hydroxymethyl)essigsäure]**
12,6 g (17,01 mmol) der Titelverbindung aus Beispiel 2a werden in einer Mischung aus 200 ml Methanol/100 ml Wasser gelöst und 4 g Palladiumkatalysator zugegeben (10 % Pd auf Aktivkohle). Man hydriert 5 Stunden bei 50°C. Es wird vom Katalysator abfiltriert und im Vakuum eingedampft. Ausbeute: 7,84 g (98 % der Theorie) eines glasigen Feststoffes (6,9 % Wasser laut Analyse).

| Analyse (auf Wasser korrigiert): | | | |
|---|---|---|---|
| C 51,06 | H 6,43 | N 11,91 | (Ber.) |
| C 50,97 | H 6,51 | N 11,81 | (Gef.) |

c) **Gadolinium-Komplex von 3,6,9,15-Tetraazabicyclo[9.3.1] pentadeca-1(15),11,13-trien-3,6,9-tris[α-(hydroxymethyl) essigsäure]**
7,5 g (15,94 mmol) der Titelverbindung aus Beispiel 2b werden in 50 ml entionisiertem Wasser gelöst und 2,89 g (7,97 mmol) Gadoliniumoxid zugesetzt. Man erwärmt 3 Stunden bei 90°C. Die abgekühlte Lösung wird mit je 2 ml saurem Ionenaustauscher (IR 120) und 2 ml basischem Austauscher (IRA 410) eine Stunde bei Raumtemperatur gerührt. Man filtriert vom Austauscher ab und kocht das Filtrat kurz mit Aktivkohle auf. Nach Filtration und Gefriertrocknung erhält man 9,56 g (96 % der Theorie) eines farblosen, amorphen Pulvers (8,1 % Wasser laut Analyse).

| Analyse (auf Wasser korrigiert): | | | | |
|---|---|---|---|---|
| C 38,45 | H 4,36 | N 8,97 | Gd 25,17 | (Ber.) |
| C 38,37 | H 4,43 | N 8,89 | Gd 25,06 | (Gef.) |

### Beispiel 3

a) **3,6,9,15-Tetraazabicyclo[9.3.1]pentadeca-1(15),11,13-trien-3,6,9-tris[α(1,2-O-isopropyliden-1,2-dihydroxyethyl)essigsäureethylester]**
Eine Mischung aus 15 g (72,71 mmol)3,6,9,15-Tetraazabicyclo[9.3.1]pentadeca-1(15),11,13-trien, 156,38 g (436,28 mmol) 3,4-O-Isopropyliden-2-(p-tolylsulfonyl)3,4-dihydroxybuttersäureethylester, 60,3 g (436,28 mmol) Kaliumcarbonat und 2,41 g (14,54 mmol) Kaliumjodid in 400 ml Acetonitril wird 48 Stunden unter Rückfluß erhitzt. Man dampft im Vakuum ein und nimmt den Rückstand mit 500 ml Methylenchlorid auf. Man extrahiert 3 mal mit 200 ml Wasser und trocknet die organische Phase über Magnesiumsulfat. Nach Eindampfen wird das zurückbleibende Öl an Kieselgel chromatographiert (Laufmittel: Methylenchlorid/Hexan/Methanol= 20:4:1)
Ausbeute: 17,24 g (31 % der Theorie) eines gelben, zähen Öls.

| Analyse: | | | |
|---|---|---|---|
| C 59,67 | H 7,91 | N 7,32 | (Ber.) |
| C 59,59 | H 7,98 | N 7,27 | (Gef.) |

b) **3,6,9,15-Tetraazybicylo[9.3.1]pentadeca-1(15),11,13-trien-3,6,9-tris[α(1,2-dihydroxyethyl)essigsäure]**
16,5 g (21,57 mmol) der Titelverbindung aus Beispiel 3a werden in 100 ml Ethanol gelöst und 50 ml 5N Natronlauge zugesetzt. Man erhitzt 10 Stunden unter Rückfluß und dampft im Vakuum ein. Der Rückstand wird in 250 ml Methanol gelöst und vom Natriumchlorid abfiltriert. Das Filtrat wird im Vakuum eingedampft und der Rückstand am Ionenaustauscher wie folgt gereinigt: Man löst in 50 ml Wasser und gibt die Lösung auf eine Kationenaustauschersäule (IR 120). Nach Spülung mit Wasser wird der Ligand mit 0,5 N wässriger Ammoniak-Lösung eluiert. Die Hauptfraktionen werden eingedampft, mit wenig Wasser aufgenommen und über eine Ionenaustauschersäule (IRA 67) gegeben. Man wäscht zuerst mit Wasser und eluiert dann mit 0,5 N Ameisensäure. Man dampft im Vakuum ein und löst den Rückstand in wenig heißem Methanol. Durch vorsichtige Zugabe von Aceton und Kühlung im Eisbad kristallisiert die Titelverbindung aus.
Ausbeute: 8,22 g (68 % der Theorie) eines glasigen Feststoffes (9,2 % Wasser laut Analyse).

| Analyse (auf Wasser korrigiert): | | | |
|---|---|---|---|
| C 49,28 | H 6,47 | N 9,99 | (Ber.) |
| C 49,17 | H 6,56 | N 9,88 | (Gef.) |

c) **Gadolinium-Komplex der 3,6,9,15-Tetraazybicylo[9.3.1]pentadeca-1(15),11,13-trien-3,6,9-tris[α(1,2-dihydroxyethyl)essigsäure]**
8 g (14,27 mmol) der Titelverbindung aus Beispiel 3b werden in 60 ml entionisiertem Wasser gelöst und 2,58 g (7,135 mmol) Gadoliniumoxid zugesetzt. Man erwärmt 3 Stunden bei 90°C. Die abgekühlte Lösung wird mit je 2 ml saurem Ionenaustauscher (IR 120) und 2 ml basischem Austauscher (IRA 410) eine Stunde bei Raumtemperatur gerührt. Man filtriert vom Austauscher ab und kocht das Filtrat mit Aktivkohle auf. Nach Filtrieren und Gefriertrocknung erhält man 9,89 g (97 % der Theorie) eines farblosen amorphen Pulvers (enthält 7,3 % Wasser laut Analyse).

| Analyse (auf Wasser korrigiert): | | | | |
|---|---|---|---|---|
| C 38,65 | H 4,65 | N 7,84 | Gd 22,00 | (Ber.) |
| C 38,54 | H 4,74 | N 7,78 | Gd 21,92 | (Gef.) |

### Beispiel 4

a) **2,2',6,6'-Tetra(hydroxymethyl)-4,4'-bipyridin**
50 g (128,77 mmol) 2,2',6,6'-Tetra(methoxycarboxyl)-4,4'-bipyridin werden in einer Mischung aus 400 ml Dioxan/400 ml Wasser gelöst und portionsweise 48,71 g (1,28 mol) Natriumborhydrid zugegeben. Man rührt über Nacht bei Raumtemperatur. Die Lösung wird mit 5 N Salzsäure angesäuert und zur Trockene eingedampft. Der Rückstand wird in 1 Liter 1 N Natronlauge suspendiert und 3 mal mit 250 ml Chloroform extrahiert. Die organischen Phasen werden über Magnesiumsulfat getrocknet und im Vakuum eingedampft. Der Rückstand wird aus Ethanol/Ether umkristallisiert.
Ausbeute: 29,53 g (83 % der Theorie) farblose Kristalle.

| Analyse: | | | |
|---|---|---|---|
| C 60,86 | H 5,84 | N 10,14 | (Ber.) |
| C 60,77 | H 5,93 | N 10,06 | (Gef.) |

b) **2,2',6,6'-Tetra(chlormethyl)-4,4'-bipyridin**
29 g (104,96 mmol) der Titelverbindung aus Beispiel 4a werden in 250 g (2,1 mol) Thionylchlorid 5 Stunden am Rückfluß erhitzt. Man dampft zur Trockene ein und nimmt den Rückstand mit 200 ml konz. Sodalösung auf. Es wird 2 mal mit 150 ml Methylenchlorid extrahiert. Die organische Phase wird über Magnesiumsulfat getrocknet und im Vakuum eingedampft. Der Rückstand wird aus Ether/Hexan kristallisiert.
Ausbeute: 35,54 g (94 % der Theorie) farblose Kristalle.

| Analyse: | | | | |
|---|---|---|---|---|
| C 48,03 | H 3,45 | N 8,08 | Gd 40,51 | (Ber.) |
| C 48,10 | H 3,40 | N 7,96 | Gd 40,59 | (Gef.) |

c) **13,13'-Bis[3,6,9-tris(p-tolylsulfonyl)-3,6,9,15-tetraazabicyclo[9.3.1]pentadeca-1(15),11,13-trien]**
Zu 118,43 g (194,25 mmol) N,N',N''-Tris(p-tolylsulfonyl)diethylentriamin-N,N''-dinatriumsalz in 1600 ml Dimethylformamid wird bei 100°C eine Lösung aus 34 g (97,12 mmol) der Titelverbindung aus Beispiel 4b (gelöst in 700 ml Dimethylformamid) innerhalb 4 Stunden zugetropft. Man läßt über Nacht bei 100°C rühren. In die heiße Lösung tropft man 2 l Wasser und läßt auf 0°C abkühlen. Der Niederschlag wird abgesaugt und mit Wasser gewaschen. Nach Trocknung im Vakuum (60°C) wird aus Acetonitril umkristallisiert. Man erhält 79,13 g (61 % der Theorie) eines cremefarbenen Pulvers.

| Analyse: | | | | |
|---|---|---|---|---|
| C 57,55 | H 5,28 | N 8,39 | S 14,40 | (Ber.) |
| C 57,47 | H 5,35 | N 8,31 | S 14,32 | (Gef.) |

d) **13,13'-Bis[3,6,9,15-tetraazabicyclo[9.3.1]pentadeca-1(15), 11,13,-trien] Oktahydrosulfat**
79 g (59,15 mmol) der Titelverbindung aus Beispiel 4c werden in 270 ml konz. Schwefelsäure eingetragen und 48 Stunden bei 100°C gerührt. Man kühlt auf 0°C und tropft 1,35 l absoluten Ether hinzu. Der Niederschlag wird abgesaugt und in 500 ml Methanol ausgerührt. Nach Abfiltrieren und Trocknen im Vakuum erhält man 65,74 g (93 % der Theorie) eines an der Luft zerlaufenden Feststoffes.

| Analyse: | | | | |
|---|---|---|---|---|
| C 22,11 | H 4,22 | N 9,38 | S 21,46 | (Ber.) |
| C 22,04 | H 4,33 | N 9,29 | S 21,38 | (Gef.) |

e) **13,13'-Bis[3,6,9,15-tetraazabicyclo[9.3.1]pentadeca-1(15), 11,13-trien]**
65,5 g (54,80 mmol) der Titelverbindung aus Beispiel 4d werden in 100 ml Wasser gelöst und der pH-Wert mit 32 %iger Natronlauge auf pH 13 gestellt. Man extrahiert 3 mal mit 250 ml heißem Toluol. Die vereinigten Toluolphasen werden mit 20 g feingepulverten Natriumhydroxid 1 Stunde unter Rückfluß erhitzt. Man filtriert und dampft das Filtrat zur Trockene ein.
Ausbeute: 21,6 g (96 % der Theorie) eines leicht gelbgefärbten Feststoffes.

| Analyse: | | | |
|---|---|---|---|
| C 64,36 | H 8,35 | N 27,29 | (Ber.) |
| C 64,27 | H 8,44 | N 27,22 | (Gef.) |

f) **13,13'-Bis[3,6,9,15-tetraazabicyclo[9.3.1]pentadeca-1(15), 11,13-trien-3,6,9-tris[α(benzyloxymethyl)essigsäure]]**
21,5 g (52,37 mmol) der Titelverbindung aus Beispiel 4e und 247,8 g (1,05 mol) des Natriumsalzes der 2-Chloro-3-benzyloxypropionsäure in 400 ml Wasser werden 48 Stunden auf 70°C erhitzt. Die Lösung wird mit 800 ml Wasser verdünnt und mit 600 ml 2 N Salzsäure versetzt. Man extrahiert 5 mal mit je 300 ml Methylenchlorid. Die Wasserphase wird im Vakuum eingedampft. Der Rückstand wird in 500 ml Ethanol gelöst und vom Natriumchlorid abfiltriert. Man dampft im Vakuum ein und chromatographiert den Rückstand an Kieselgel (Laufmittel: Ethanol/Wasser= 20:1) Die Hauptfraktionen werden im Vakuum eingedampft und in 100 ml 5 %iger Salzsäure gelöst. Die Lösung wird auf eine Säule, gefüllt mit Reillex^{R} (= Poly-4-vinylpyridin) gegeben und das Produkt mit einer Mischung aus Wasser/Methanol 2:1 eluiert. Nach Eindampfen der Hauptfraktionen erhält man 20,92 g (27 % der Theorie) eines stark hygroskopischen Feststoffes (8,1 % Wasser laut Analyse).

| Analyse (auf Wasser korrigiert): | | | |
|---|---|---|---|
| C 66,56 | H 6,40 | N 7,57 | (Ber.) |
| C 66,47 | H 6,51 | N 7,48 | (Gef.) |

g) **13,13'-Bis[3,6,9,15-tetraazabicyclo[9.3.1]pentadeca-1(15), 11,13-trien-3,6,9-tris[α(hydroxymethyl)essigsäure]]**
20,5 g (13,85 mmol) der Titelverbindung aus Beispiel 4f werden in einer Mischung aus 300 ml Methanol/150 ml Wasser gelöst und 7 g Palladium-Katalysator (10 % Pd auf Aktivkohle) zugegeben. Man hydriert 5 Stunden bei 50°C. Es wird vom Katalysator abfiltriert und im Vakuum eingedampft.
Ausbeute: 12,62 g (97 % der Theorie) eines glasigen Feststoffes (8,5 % Wasser laut Analyse).

| Analyse (auf Wasser korrigiert): | | | |
|---|---|---|---|
| C 51,17 | H 6,23 | N 11,93 | (Ber.) |
| C 51,07 | H 6,31 | N 11,87 | (Gef.) |

h) **Gadolinium-Komplex der 13,13'-Bis[3,6,9,15-tetraazabicyclo-[9.3.1]pentadeca-1(15),11,13-trien-3,6,9-tris[α(hydroxymethyl)essigsäure]]**
12 g (12,78 mmol) der Titelverbindung aus Beispiel 4g werden in 80 ml entionisiertem Wasser gelöst und 4,63 g (12,78 mmol) Gadoliniumoxid zugesetzt. Man erwärmt 3 Stunden bei 90°C. Die abgefüllte Lösung wird mit je 5 ml saurem Ionenaustauscher (IR 120) und 5 ml basischem Austauscher (IRA 410) eine Stunde bei Raumtemperatur gerührt. Man filtriert vom Austauscher ab und kocht das Filtrat kurz mit Aktivkohle auf. Nach Filtrieren und Gefriertrocknung erhält man 15,3 g (96 % der Theorie) eines farblosen, amorphen Pulvers (enthält 9,3 % Wasser laut Analyse).

| Analyse (auf Wasser korrigiert): | | | | |
|---|---|---|---|---|
| C 38,52 | H 4,20 | N 8,98 | Gd 25,21 | (Ber.) |
| C 38,46 | H 4,28 | N 8,91 | Gd 25,14 | (Gef.) |

### Beispiel 5

a) **Trans-5-(p-Tolylsulfonyl)-amino-6-(p-tolylsulfonyloxy)-2,2-dimethyl-1,3-dioxepan**
Zu einer Lösung von 100 g trans-6-Amino-2,3-dimethyl-1,3-dioxepan-5-ol in 903 ml Pyridin gibt man unter Rühren bei -5° bis 0°C 295,67 g p-Toluolsulfochlorid in Portionen. Man läßt 72 Stunden bei +4°C stehen und rührt die Mischung dann in 10 l Eiswasser ein. Nach Absaugen und Waschen des Niederschlags mit Wasser trocknet man den Rückstand im Trockenschrank bei 50° und 200 Torr für 48 Stunden. Zur Reinigung wird das Rohprodukt aus 5 l Dioxan umkrist. Man erhält 196 g der Titelverbindung als weißes Pulver.
F. 200-202°C.
b) **Mononatriumsalz von N-[2-(N-Tolylsulfonylamino)-ethyl]-p-tolylsulfonamid**
Man suspendiert 150 g N-[2-(N-Tolylsulfonylamino)-ethyl]-p-tolylsulfonylamid in 1,25 l Ethanol, erhitzt unter Rückfluß und tropft eine Lösung von 10,3 g Natrium in 300 ml Ethanol zu, wobei eine Lösung entsteht. Beim Abkühlen fällt die Titelverbindung aus. Man saugt ab, wäscht den Niederschlag mit Ethanol und trocknet bei 50°C und 200 Torr. Man erhält 119 g der Titelverbindung als weißes Pulver.
c) **Cis-2,2-Dimethyl-5-[N-(p-tolylsulfonyl)-amino]-6-[N-(p-tolylsulfonyl)-N-(N'-2-p-tolylsulfonylaminomethyl)]-1,3-dioxepan**
116 g des Mononatriumsalzes aus Beispiel 5b werden in 2,66 l Dimethylformamid suspendiert. Bei 100°C tropft man eine Lösung von 141 g trans-5-(p-Tolylsulfonyl)-amino-6-(p-tolylsulfonyloxy)-2,2-dimethyl-1,3-dioxepan in 1,5 l Dimethylformamid zu und rührt 5 Stunden bei 120°C Badtemperatur. Die Reaktionslösung wird dann im Vakuum auf 1 l eingeengt und mit 10 l Eiswasser verdünnt. Man saugt ab, wäscht den Niederschlag mit Wasser und trocknet bei 50°C und 200 Torr. Man erhält 182 g der rohen Titelverbindung, die zur Reinigung mit 1,85 l Ethanol ausgekocht wird. Nach Absaugen und Trocknen erhält man 125 g der Titelverbindung als weißes Pulver.
F. 190-194°C.
d) **Dinatriumsalz von cis-2,2-Dimethyl-5-[N-(p-tolylsulfonyl)-amino]-6-[N-(p-tolylsulfonyl)-N-(N'-2-p-tolylsulfonylaminomethyl)]-1,3-dioxepan**
Man suspendiert 87,8 g der nach Beispiel 5c hergestellten Substanz in 410 ml Ethanol, erhitzt zum Sieden und tropft eine Lösung von 6,67 g Natrium in 200 ml Ethanol zu. Man kühlt im Eisbad ab, versetzt mit 450 ml Ether und saugt vom ausgefallenen Niederschlag ab, der bei 80°C und 200 Torr getrocknet wird. Man erhält 91 g der Titelverbindung als weißes Pulver.
e) **Acetonid von 4,5-Bis(hydroxymethyl)-3,6,9-tritosylsulfonyl-3,6,9,15-tetraazabicyclo[9.3.1]pentadeca-1(15),11,13-trien**
Man löst 78,75 g des Dinatriumsalzes aus Beispiel 5d in 880 ml Dimethylformamid, erhitzt auf 100°C und tropft eine Lösung von 19,53 g Bis-(2,6-chlormethyl)-pyridin in 360 ml Dimethylformamid zu, erhitzt noch 5 Stunden auf 120°C und engt im Vakuum auf 300 ml ein. Die Lösung wird in 5 l Eiswasser eingerührt, der ausgefallene Niederschlag abgesaugt, mit Wasser gewaschen und getrocknet. Das Rohprodukt kristallisiert man aus 700 ml Dioxan um und erhält 45 g der Titelverbindung als weißes Pulver.
F. 244-250°C.
f) **Acetonid vom 4,5-Bis(hydroxymethyl)-3,6,9,15-tetraazabicyclo [9.3.1]pentadeca-1(15),11,13-trien**
Zu 260 ml flüssigem Ammoniak gibt man eine Suspension von 20 g der nach Beispiel 5e erhaltenen Substanz in 140 ml Tetrahydrofuran, rührt im Kältebad bei -50°C und trägt in Portionen insgesamt 14,4 g Natrium ein. Man rührt noch 5 Stunden bei -60°C, entfert dann das Kältebad und tropft 50 ml Ethanol zu und läßt den Ammoniak verdampfen, dampft im Vakuum zur Trockene und reinigt den Rückstand durch Chromatographie an Kieselgel. Man eluiert mit Chloroform/Ethanol/Conc. Ammoniaklösung (3/1/0,5) und erhält 5,30 g der Titelverbindung als Öl.

| Analyse: | | | |
|---|---|---|---|
| C 62,72 | H 8,55 | N 18,29 | (Ber.) |
| C 62,51 | H 8,41 | N 18,45 | (Gef.) |

g) **Acetonid von 4,5-Bis(hydroxymethyl)-3,6,9,15-tetraazabicyclo [9.3.1]pentadeca-1(15),11,13-trien-3,6,9-tris(essigsäure-tert.butylester)**
Zu einer Lösung von 4 g der nach Beispiel 5f hergestellten Substanz in 100 ml Tetrahydrofuran und 10 ml Wasser gibt man 5,51 g wasserfreies Natriumcarbonat und 10,2 g Bromessigsäure-tert.-butylester und rührt 5 Stunden bei 50°C. Man filtriert, dampft im Vakuum ein und rührt den öligen Rückstand mit 50 ml Hexan und dekantiert. Der Rückstand wird durch Chromatographie an 100 g Kieselgel mit Dichlormethan (1-10 % Ethanol)gereinigt. Man erhält 5,7 g der Titelverbindung als hellgelbes Öl.
h) **4,5-Bis(hydroxymethyl)-3,6,9,15-tetraazabicyclo[9.3.1] pentadeca-1(15),11,13-trien-3,6,9-tris-essigsäure**
Eine Mischung von 5,3 g des nach Beispiel 5g hergestellten Esters und 50 ml Trifluoressigsäure werden 3 Stunden bei 50°C gerührt. Man gibt dann 10 ml Wasser zu, rührt weitere 2 Stunden bei 50°C und dampft dann im Vakuum zur Trockene. Den Rückstand löst man in 20 ml Wasser und läßt die Lösung über eine Säule mit 100 ml Reillex (poly-4-vinyl-pyridin) laufen, eluiert mit 100 ml Wasser und dampft das Eluat im Vakuum ein. Man erhält ein amorphes Pulver, das noch 8,5 % Wasser enthält. Ausbeute 2,90 g

| Analyse: | | | |
|---|---|---|---|
| C 51,81 | H 6,41 | N 12,72 | (Ber.) |
| C 51,63 | H 6,70 | N 12,51 | (Gef.) |

i) **Gadoliniumkomplex der 4,5-Bis(hydroxymethyl)-3,6,9,15-tetraazabicyclo[9.3.1]pentadeca-1(15),11,13-trien-3,6,9-tris-essigsäure**
2 g (Wassergehalt 8,5 %, entspricht 1,83 g = 4,29 mMol) 4,5-Bis(hydroxymethyl)-3,6,9,15-tetraazabicyclo[9.3.1] pentadeca-1(15),11,13-trien-3,6,9-tris-essigsäure (Herstellung s. Beispiel 5h) und 778 mg Gadoliniumoxid werden 5 Stunden mit 50 ml Wasser bei 90°C gerührt. Nach dem Abkühlen rührt man nacheinander mit je 10 ml Anionenaustauscher IRA-410 und Kationenaustauscher IRC-50, filtriert und unterwirft die Lösung einer Gefriertrocknung. Man erhält 2,35 g der Titelverbindung als lockeres weißes Pulver, Wassergehalt nach K.-Fischer-Titration: 7,3 %.

| Analyse (nach Korrektur des Wassergehalts): C₁₉H₂₅GdN₄O₈ | | | | |
|---|---|---|---|---|
| C 38,38 | H 4,24 | N 9,42 | Gd 26,44 | (Ber.) |
| C 38,51 | H 4,31 | N 9,36 | Gd 26,19 | (Gef.) |

### Beispiel 6

a) **Acetonid von 4,5-Bis(hydroxymethyl)-3,6,9-tris-[dihydro-2 (3H)-furanon-3-yl]-3,6,9,15-tetraazabicyclo[9.3.1]pentadeca-1(15),11,13-trien**
Zu einer Lösung von 5 g (16,34 mMol) Acetonid von 4,5-Bis (hydroxymethyl)-3,6,9,15-tetraazabicyclo[9.3.1]pentadeca-1(15),11,13-trien in 100 ml Acetonitril gibt man 12 g Kaliumcarbonat, 260 mg Kaliumjodid und 13,50 g α-Brom-γ-butyrolacton und erhitzt 48 Stunden zum Sieden. Man dampft dann im Vakuum ein, löst den Rückstand in Methylenchlorid, schüttelt mehrmals mit Wasser, trocknet die organische Phase über Natriumsulfat und dampft im Vakuum ein. Der ölige Rückstand wird mit Methylenchlorid/Methanol (15:1) an 150 g Kieselgel chromatographiert. Man erhält 5,3 g der Titelverbindung als hellgelbes zähes Öl.
b) **4,5-Bis(hydroxymethyl)-3,6,9,15-tetraazabicyclo[9.3.1] pentadeca-1(15),11,13-trien-3,6,9-tris-[α-(2-hydroxyethyl)]-essigsäure**
5 g der nach Beispiel 6a hergestellten Verbindung werden in 50 ml Wasser gelöst und durch Zugabe von Salzsäure auf einen pH-Wert von 2 eingestellt. Man erhitzt 5 Stunden unter Rückfluß, kühlt auf Raumtemperatur ab und läßt die Lösung über eine Säule mit 10 g Reillex (Poly-4-vinylpyridin) laufen, wäscht die Säule mit 20 ml Wasser nach und unterwirft die vereinigten Eluate einer Gefriertrocknung. Man erhält 4,05 g der Titelverbindung als lockeres Pulver mit einem Wassergehalt von 7,2 %.

| Analyse (nach Korrektur des Wassergehalts) : C₂₅H₄₀N₄O₁₁ | | | |
|---|---|---|---|
| C 52,44 | H 7,04 | N 9,78 | (Ber.) |
| C 52,61 | H 7,33 | N 9,62 | (Gef.) |

c) **Gadoliniumkomplex der 4,5-Bis(hydroxymethyl)-3,6,9,15-tetraazabicyclo[9.3.1]pentadeca-1(15),11,13-trien-3,6,9-tris-[α-(2-hydroxyethyl)]-essigsäure**
1,50 g (2,68 mMol) 4,5-Bis(hydroxymethyl)-3,6,9,15-tetraazabicyclo[9.3.1]pentadeca-1(15),11,13-trien-3,6,9-tris-[α-(2-hydroxyethyl)]-essigsäure werden in 25 ml Wasser mit 487 mg Gadoliniumoxid 4 Stunden bei 90°C gerührt. Nach dem Abkühlen wird die Lösung nacheinander mit 5 ml Anionenaustauscher IRA-410 und 5 ml Kationenaustauscher IRC-50 gerührt, filtriert und einer Gefriertrocknung unterworfen. Man erhält 1,69 g der Titelverbindung als weißes Pulver mit einem Wassergehalt von 4,3 %.

| Analyse (nach Korrektur des Wassergehalts) : C₂₅H₃₇GdN₄O₁₁ | | | | |
|---|---|---|---|---|
| C 41,31 | H 5,13 | N 7,71 | Gd 21,63 | (Ber.) |
| C 41,07 | H 5,33 | N 7,61 | Gd 21,89 | (Gef.) |

### Beispiel 7

a) **4-Hydroxymethyl-3,6,9-tritosyl-3,6,9,15-tetraazabicyclo [9.3.1]pentadeca-1(15),11,13-trien**
Zu einer Lösung von 59,57 g 3-Aza-1-hydroxymethyl-1,3,5-tritosyl-pentandiamin (Herstellung siehe Internationale Patentanmeldung PCT/DE 88/00200, WO 88/08422, Seite 45) in 500 ml Dimethylformamid gibt man in Portionen 9,60 g einer 50 %igen Suspension von Natriumhydrid in Mineralöl und erhitzt 1 Stunde auf 80°C. Zu dieser Lösung tropft man 17,61 g 2,6-Bis(chlormethyl)pyridin gelöst in 150 ml Dimethylformamid und erhitzt 6 Stunden auf 110°C. Man engt im Vakuum auf ca. 220 ml ein und tropft 1 l Wasser zu, saugt den Niederschlag ab, wäscht mit Wasser und trocknet über Nacht bei 50°C und 200 mbar. Das Rohprodukt wird aus 500 ml Ethanol umkristallisiert und liefert 45 g der Titelverbindung als gelben Feststoff.
b) **4-Hydroxymethyl-3,6,9,15-tetraazabicyclo[9.3.1]pentadeca-1(15),11,13-trien**
42 g der nach Beispiel 7a erhaltenen Verbindung werden mit 120 ml conc. Schwefelsäure 48 Stunden auf 100 °C erhitzt. Man kühlt auf 0°C ab und tropft 350 ml Diethylether zu. Das Salz der Titelverbindung fällt dabei aus. Man saugt ab, löst den Rückstand in 100 ml Wasser und versetzt mit 40 g Natriumhydroxid und extrahiert mehrmals mit Dichlormethan. Die vereinigten organischen Phasen werden über Natriumsulfat getrocknet und im Vakuum eingedampft. Man erhält 13,3 g der Titelverbindung als zähes Öl.
c) **4-Hydroxymethyl-3,6,9,15-tetraazabicyclo[9.3.1]pentadeca -1(15),11,13-trien-3,6,9-triessigsäure**
10 g des nach Beispiel 7b hergestellten Amins werden in 100 ml Wasser gelöst und mit 13,21 g Chloressigsäure versetzt. Man rührt 5 Stunden bei 60°C und hält den pH-Wert während dieser Zeit durch Zugabe von 10N Natronlauge bei 9,0. Man kühlt auf 0°C, versetzt mit 100 ml Ethanol und säuert mit conc. Salzsäure auf pH 1 an, saugt den ausgefallenen Niederschlag ab, löst in 50 ml Wasser und gibt die Lösung über eine Säule mit 30 ml Reillex (Poly-4-vinylpyridin), wäscht mit 50 ml Wasser nach, vereinigt die Eluate und unterwirft sie einer Gefriertrocknung. Man erhält 16,8 g der Titelverbindung als amorphes Pulver mit einem Wassergehalt von 9,3 %.

| Analyse (nach Korrektur des Wassergehalts): C₁₈H₂₆N₄O₇ | | | |
|---|---|---|---|
| C 52,68 | H 6,39 | N 13,65 | (Ber.) |
| C 52,49 | H 6,54 | N 13,81 | (Gef.) |

d) **Gadoliniumkomplex der 4-Hydroxymethyl-3,6,9,15-tetraazabicyclo[9.3.1]pentadeca-1(15),11,13-trien-3,6,9-triessigsäure**
Zu einer Lösung von 2,3 g 4-Hydroxymethyl-3,6,9,15-tetraazabicyclo[9.3.1]pentadeca-1(15),11,13-trien in 30 ml Wasser gibt man 1,015 g Gadoliniumoxid und erhitzt 1 Stunde auf 90°C. Man filtriert von wenig nicht umgesetzten Oxid ab und läßt die Lösung nacheinander über je 10 ml Anionenaustauscher IRA-410 und Kationenaustauscher IRC 50 laufen, wäscht mit 30 ml Wasser nach und unterwirft die vereinigten Eluate einer Gefriertrocknung. Man erhält 3,05 g der Titelverbindung als Pulver mit einem Wassergehalt von 7,5 %.

| Analyse (nach Korrektur des Wassergehalts): | | | | |
|---|---|---|---|---|
| C 38,29 | H 4,11 | N 9,92 | Gd 27,85 | (Ber.) |
| C 38,44 | H 4,32 | N 9,68 | Gd 27,71 | (Gef.) |

### Beispiel 8

a) **4-Benzyloxymethyl-3,6,9,15-tetraazabicyclo[9.3.1lpentadeca-1(15),11,13-trien-3,6,9-triessigsäure**
Zu einer Lösung von 10 g (≙ 24,36 mMol) 4-Hydroxymethyl-3,6,9,15-tetraazabicyclo[9.3.1]pentadeca-1(15),11,13-trien-3,6,9,triessigsäure (hergestellt nach Beispiel 7b) in 150 ml Dimethylformamid gibt man 0,5 g Kaliumjodid, 4,17 g Benzylbromid und 5 g Natriumcarbonat. Man erhitzt 20 Stunden auf 60°C, engt im Vakuum ein, versetzt mit 100 ml Wasser und 300 ml Ethanol und stellt den pH-Wert durch Zugabe von conc. Salzsäure auf 2 ein. Man saugt vom ausgefallenen Niederschlag ab, löst den Niederschlag in 100 ml Wasser und gibt die Lösung über eine Säule mit 50 g Reillex (Poly -4-vinylpyridin), wäscht die Säule mit 50 ml Wasser und unterwirft die vereinigten wässrigen Phasen einer Gefriertrocknung. Man erhält 8 g der Titelverbindung als amorphes Pulver.

| Analyse: | | | |
|---|---|---|---|
| C 59,99 | H 6,44 | N 11,19 | (Ber.) |
| C 59,71 | H 6,49 | N 11,38 | (Gef.) |

b) **Gadoliniumkomplex der 4-Benzyloxymethyl-3,6,9,15-tetraazabicyclo[9.3.1]pentadeca-1(15),11,13-trien-3,6,9-triessigsäure**
Zu einer Lösung von 5 g 4-Benzyloxymethyl-3,6,9,15-tetraazabicyclo[9.3.1]pentadeca-1(15),11,13-trien-3,6,9-triessigsäure in 100 ml Wasser gibt man 1,81 g Gadoliniumoxid, erhitzt 3 Stunden auf 80-90°C, filtriert und läßt die Lösung nacheinander über Säulen mit je 15 ml Anionenaustauscher IRA-410 und Kationenaustauscher IRC-50 laufen, wäscht die Säulen mit 75 ml Wasser nach und unterwirft die vereinigten wässrigen Phasen einer Gefriertrocknung. Man erhält 5,85 g der Titelverbindung als amorphes Pulver, Wassergehalt 7,4 %.

| Analyse (nach Korrektur des Wassergehalts): | | | | |
|---|---|---|---|---|
| C 45,86 | H 4,46 | N 8,56 | Gd 24,02 | (Ber.) |
| C 45,69 | H 4,71 | N 8,72 | Gd 23,81 | (Gef.) |

### Beispiel 9

### Gadolinium-Komplex der 3,6,9,15-Tetraazabicyclo[9.3.1] pentadecan-3,6,9-tris[α(hydroxymethyl)essigsäure]

4,5 g (7,2 mmol) der Titelverbindung aus Beispiel 2c werden in 150 ml entionisiertem Wasser gelöst und in einem Autoklaven über einen Rhodium-Katalysator (5 % Rh/C) bei 30 Bar und 40 °C hydriert. Nach 12 Stunden filtriert man vom Katalysator ab und rührt das Filtrat mit je 3 ml Kationenaustauscher (IR 120) und 3 ml Anionenaustauscher (IRA 410) eine Stunde lang. Es wird vom Austauscher filtriert und gefriergetrocknet.
Ausbeute: 4,18 g (92 % der Theorie) eines farblosen, amorphen Pulvers (enthält 6,7 % Wasser laut Analyse).

| Analyse (auf Wasser korrigiert): | | | | |
|---|---|---|---|---|
| C 30,08 | H 5,27 | N 8,88 | Gd 24,93 | (Ber.) |
| C 30,01 | H 5,34 | N 8,78 | Gd 24,86 | (Gef.) |

### Beispiel 10

### Herstellung einer Lösung vom Gadolinium(III)-Komplex der 3,6,9,15-Tetraazabicyclo[9.3.1]pentadeca-1(15),11,13-trien-3,6,9-tris[α-(2-hydroxyethyl)essigsäure]

a) 361 g (0,5 Mol) des nach Beispiel 1b erhaltenen Komplexes (Wassergehalt: 8,3 %) werden in 500 ml Wasser pro injectione (p.i.) unter schwachem Erwärmen gelöst. Nach Zugabe von 0,8 g Tromethamin wird die Lösung mit Wasser p.i. auf 1000 ml aufgefüllt. Die Lösung wird ultrafiltriert und in Flaschen abgefüllt. Nach Hitzesterilisation ist sie für die parenterale Applikation zur Diagnostik gebrauchsfertig.
b) Die unter Beispiel 10a erhaltene ultrafiltrierte Lösung wird in Multivials steril abgefüllt und lyophilisiert. Nach Zugabe der gewünschten Menge Wasser p.i. erhält man die für die intrastitielle Injektion zur Strahlentherapie geeignete Applikationsdosis.

## Patentansprüche

1. Makrocyclische Verbindungen der allgemeinen Formel I 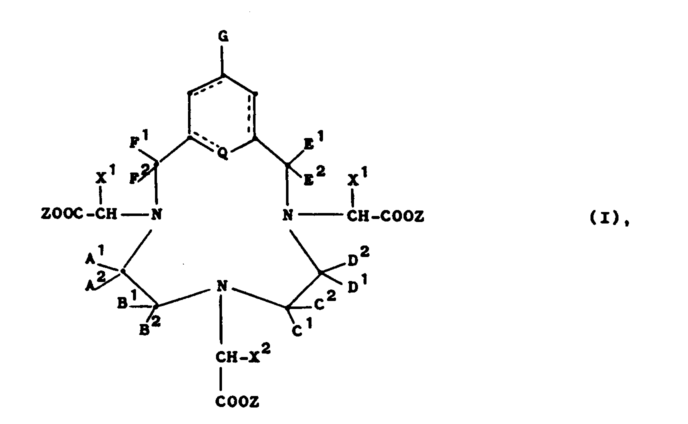 worin
... für eine Einfach- oder Doppelbindung,
Q für ein Stickstoffatom oder den Rest NH,
X¹ für ein Wasserstoffatom, eine -(CH₂)ₙ-R¹- oder 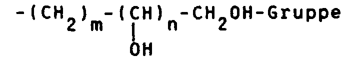 mit
n in der Bedeutung der Ziffern 1 bis 5,
m in der Bedeutung der Ziffern 0 bis 2 und
R¹ in der Bedeutung eines Wasserstoffatoms oder einer Hydroxygruppe,
X² für X¹ oder eine -(CH₂)ₙ-(O)ₗ-(CH₂)ₖ-(C₆H₄)_{q}-R²-Gruppe mit
k in der Bedeutung der Ziffern 0 bis 4,
l und q in der Bedeutung der Ziffern 0 oder 1 und
R² in der Bedeutung eines Wasserstoffatoms, einer C₁-C₄-Alkoxygruppe, einer funktionellen Gruppe oder gebunden über diese funktionelle Gruppe eines Bio- oder Makromoleküls,
A¹, A², B¹, B², C¹, C², D¹, D², E¹, E², F¹ und F² unabhängig voneinander jeweils für X²
G für R² oder einen über K gebundenen zweiten Makrocyclus der allgemeinen Formel II 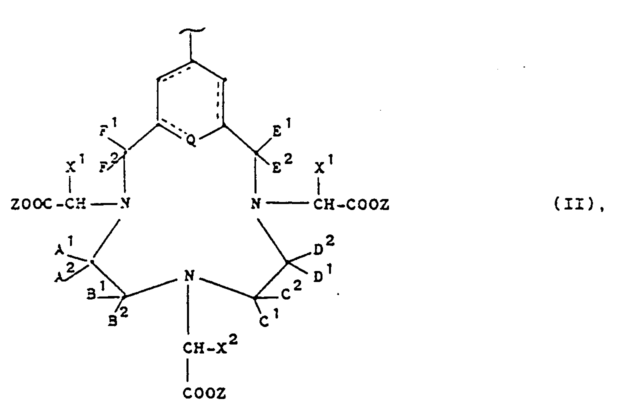 mit K in der Bedeutung einer direkten Bindung, einer Bis(carbonylamino)-gruppe (-NH-CO-CO-NH-) oder einer C₁-C₁₄-Alkylengruppe, die gegebenenfalls an den Enden Carbonyl-(>CO) oder Carbonylamino (-NH-CO-)-gruppen oder Sauerstoffatome trägt und gegebenenfalls ein oder mehrere Sauerstoffatom(e), Hydroxymethylen- (-CH-OH-) gruppe (n), CH(X²)COOZ-, acyl- oder hydroxyacylsubstituierte Iminogruppen oder ein bis zwei C-C-Doppel- und/oder C-C-Dreifachbindungen enthält,
Z für ein Wasserstoffatom und/oder ein Metallionenäquivalent eines Elements der Ordnungszahlen 21-29, 31, 32, 37-39, 42-44, 49 oder 57-83
stehen mit der Maßgabe, daß die 12 Ringsubstituenten A¹ bis F² für mindestens 8 Wasserstoffatome stehen, daß X¹ und X² nur dann gleichzeitig für Wasserstoffatome stehen, wenn mindestens einer der Ringsubstituenten A¹ bis F² nicht für ein Wasserstoffatom, A¹ bis D² nicht für (CH₂)₁₋₆-H und E¹ bis F² (CH₂)₁₋₅-H steht und daß der Makrocyclus der allgemeinen Formel I nicht mehr als ein Bio- oder Makromolekül enthält und daß gewünschtenfalls der Rest der CO₂H-Gruppen als Ester oder Amid vorliegt, sowie deren Salze mit anorganischen und/oder organischen Basen, Aminosäuren oder Aminosäureamiden.

2. Makrocyclische Verbindungen der allgemeinen Formel III 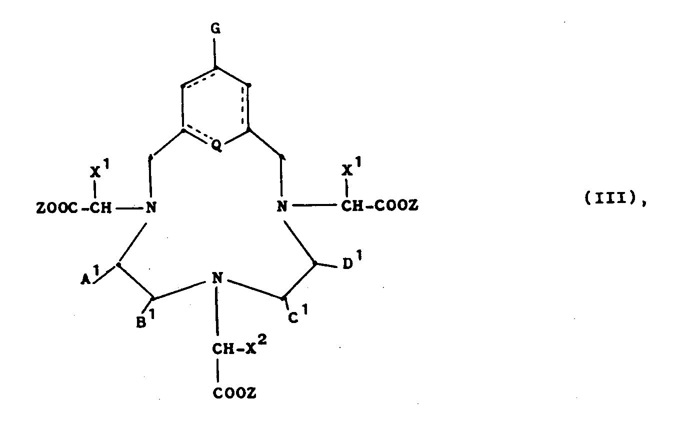 worin
... für eine Einfach- oder Doppelbindung,
Q für ein Stickstoffatom oder den Rest NH,
X¹ für ein Wasserstoffatom, eine -(CH₂)ₙ-R¹- oder 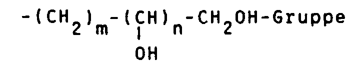 mit
n in der Bedeutung der Ziffern 1 bis 5,
m in der Bedeutung der Ziffern 0 bis 2 und
R¹ in der Bedeutung eines Wasserstoffatoms oder einer Hydroxygruppe,
X² für X¹ oder eine -(CH₂)ₙ-(O)ₗ-(CH₂)ₖ-(C₆H₄)_{q}-R²-Gruppe mit
k in der Bedeutung der Ziffern 0 bis 4,
l und q in der Bedeutung der Ziffern 0 oder 1 und
R² in der Bedeutung eines Wasserstoffatoms, einer C₁-C₄-Alkoxygruppe, einer funktionellen Gruppe oder gebunden über diese funktionelle Gruppe eines Bio- oder Makromoleküls,
A¹, B¹, C¹ und D¹ unabhängig voneinander jeweils für X²
G für R² oder einen über K gebundenen zweiten Makrocyclus der allgemeinen Formel IV 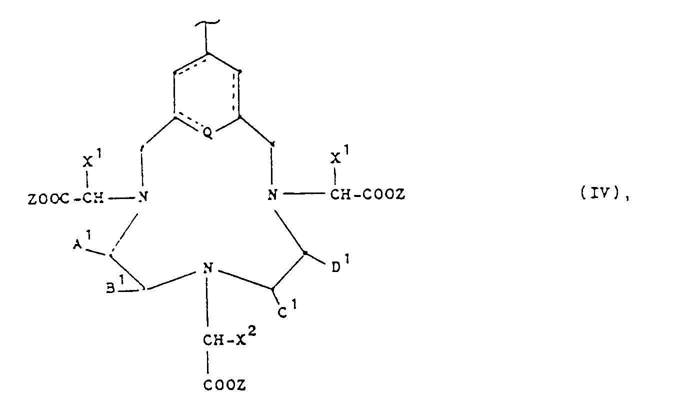 mit K in der Bedeutung einer direkten Bindung, einer Bis(carbonylamino)-gruppe (-NH-CO-CO-NH-) oder einer C₁-C₁₄-Alkylengruppe, die gegebenenfalls an den Enden Carbonyl-(>CO) oder Carbonylamino (-NH-CO-)-gruppen oder Sauerstoffatome trägt und gegebenenfalls ein oder mehrere Sauerstoffatom(e), Hydroxymethylen- (-CH-OH-) gruppe(n) CH(X²)COOZ-, acyl- oder hydroxyacylsubstituierte Iminogruppen oder ein bis zwei C-C-Doppel- und/oder C-C-Dreifachbindungen enthält,
Z für ein Wasserstoffatom und/oder ein Metallionenäquivalent eines Elements der Ordnungszahlen 21-29, 31, 32, 37-39, 42-44, 49 oder 57-83
stehen mit der Maßgabe, daß X¹ und X² nur dann gleichzeitig für Wasserstoffatome stehen, wenn mindestens einer der 4 Ringsubstituenten A¹, B¹, C¹ und D¹ nicht für ein Wasserstoffatom oder für (CH₂)₁₋₆-H steht, und daß gewünschtenfalls der Rest der CO₂H-Gruppen als Ester oder Amid vorliegt, sowie deren Salze mit anorganischen und/oder organischen Basen, Aminosäuren oder Aminosäureamiden.

3. Verbindungen gemäß Anspruch 1, dadurch gekennzeichnet, daß Z für Wasserstoffatome steht.

4. Verbindungen gemäß Anspruch 1, dadurch gekennzeichnet, daß mindestens 2 der Substituenten Z Metallionenäquivalente mindestens eines Elements der Ordnungszahlen 21-29, 42, 44 oder 57-83 oder mindestens eines Radionuklids eines Elements der Ordnungszahlen 27, 29, 31, 32, 37-39, 43, 49, 62, 64, 70 oder 77 sind.

5. Verbindungen gemäß Anspruch 1, dadurch gekennzeichnet, daß K für eine 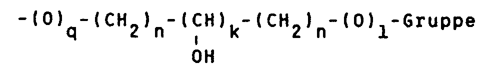 oder für eine direkte Bindung steht.

6. Verbindungen gemäß Anspruch 1, dadurch gekennzeichnet, daß die für R² stehende funktionelle Gruppe NCS, NO₂, OH, NHNH₂, NH₂, NHCOCH₂Br, NHCOCH₂Cl, CO₂H, CON₃ bedeutet.

7. Verbindungen gemäß Anspruch 1, dadurch gekennzeichnet, daß das in R² gegebenenfalls enthaltene Bio- oder Makromolekül ein Antikörper oder Antikörperfragment ist.

8. Verbindungen gemäß Anspruch 1, dadurch gekennzeichnet, daß das in R² gegebenenfalls enthaltene Bio- oder Makromolekül ein Protein wie Albumin, Globulin oder Lectin ist.

9. Verbindungen gemäß Anspruch 1, dadurch gekennzeichnet, daß das in R² gegebenenfalls enthaltene Bio- oder Makromolekül ein Polysaccharid wie Stärke, Dextran oder Dextrin ist.

10. Verbindungen gemäß Anspruch 1, dadurch gekennzeichnet, daß X¹ für CH₂OH, CH₂CH₂OH, CHOHCH₂OH steht.

11. Verbindungen gemäß Anspruch 2, dadurch gekennzeichnet, daß X², A¹, B¹, C¹ und/oder D¹ für CH₂OH, CH₂CH₂OH, CH₂OCH₂C₆H₅, CHOHCH₂OH, CH₂C₆H₄OCH₃, CH₂C₆H₅, CH₂C₆H₄O(CH₂)₃COOH, CH₂C₆H₄NCS stehen.

12. Verwendung von mindestens einer physiologisch verträglichen Verbindung gemäß Anspruch 4 für die Herstellung von Mitteln für die NMR-, Röntgen-, Radio-Diagnostik, Radioimmuno- oder Strahlen-Therapie.

13. Verfahren zur Herstellung von makrocyclischen Verbindungen der allgemeinen Formel I 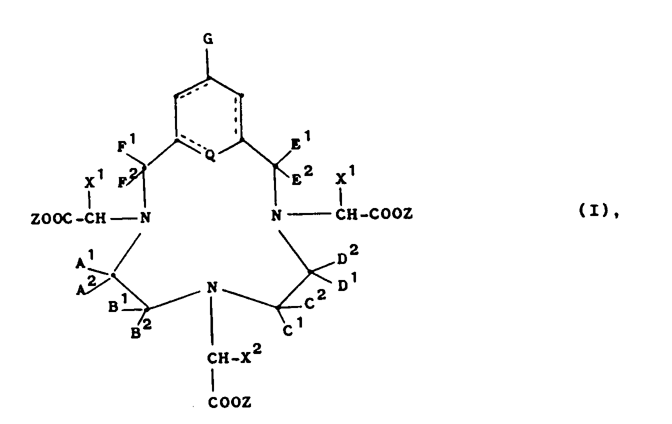 worin
... für eine Einfach- oder Doppelbindung,
Q für ein Stickstoffatom oder den Rest NH,
X¹ für ein Wasserstoffatom, eine -(CH₂)ₙ-R¹- oder 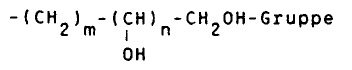 mit
n in der Bedeutung der Ziffern 1 bis 5,
m in der Bedeutung der Ziffern 0 bis 2 und
R¹ in der Bedeutung eines Wasserstoffatoms oder einer Hydroxygruppe,
X² für X¹ oder eine -(CH₂)ₙ-(O)ₗ-(CH₂)ₖ-(C₆H₄)_{q}-R²-Gruppe mit
k in der Bedeutung der Ziffern 0 bis 4,
l und q in der Bedeutung der Ziffern 0 oder 1 und
R² in der Bedeutung eines Wasserstoffatoms, einer C₁-C₄-Alkoxygruppe, einer funktionellen Gruppe oder gebunden über diese funktionelle Gruppe eines Bio- oder Makromoleküls,
A¹, A², B¹, B², C¹, C², D¹, D², E¹, E², F¹ und F² unabhängig voneinander jeweils für X²
G für R² oder einen über K gebundenen zweiten Makrocyclus der allgemeinen Formel II 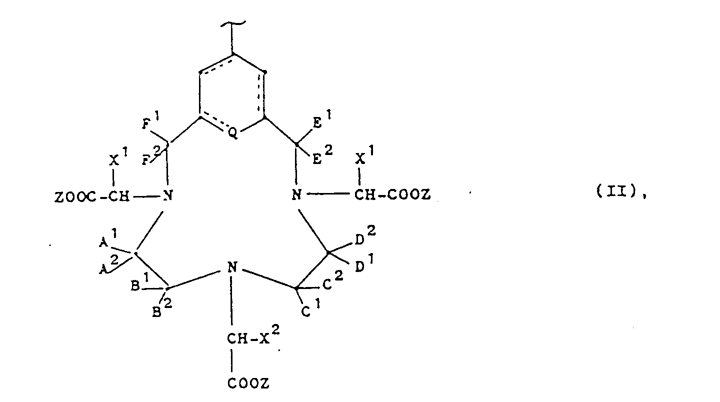 mit K in der Bedeutung einer direkten Bindung, einer Bis(carbonylamino)-gruppe (-NH-CO-CO-NH-) oder einer C₁-C₁₄-Alkylengruppe, die gegebenenfalls an den Enden Carbonyl-(>CO) oder Carbonylamino (-NH-CO-)-gruppen oder Sauerstoffatome trägt und gegebenenfalls ein oder mehrere Sauerstoffatom(e), Hydroxymethylen- (-CH-OH-) gruppe(n) CH(X² )COOZ-, acyl- oder hydroxyacylsubstituierte Iminogruppen oder ein bis zwei C-C-Doppel- und/oder C-C-Dreifachbindungen enthält,
Z für ein Wasserstoffatom und/oder ein Metallionenäquivalent eines Elements der Ordnungszahlen 21-29, 31, 32, 37-39, 42-44, 49 oder 57-83
stehen mit der Maßgabe, daß die 12 Ringsubstituenten A¹ bis F² für mindestens 8 Wasserstoffatome stehen, daß X¹ und X² nur dann gleichzeitig für Wasserstoffatome stehen, wenn mindestens einer der Ringsubstituenten A¹ bis F² nicht für ein Wasserstoffatom, A¹ bis D² nicht für (CH₂)₁₋₆-H und E¹ bis F² nicht für (CH₂)₁₋₅-H steht und daß der Makrocyclus der allgemeinen Formel I nicht mehr als ein Bio- oder Makromolekül enthält, sowie deren Salze mit anorganischen und/oder organischen Basen und daß gewünschtenfalls der Rest der CO₂H-Gruppen als Ester oder Amid vorliegt, Aminosäuren oder Aminosäureamiden, dadurch gekennzeichnet, daß man in an sich bekannter Weise in Verbindungen der allgemeinen Formel I' 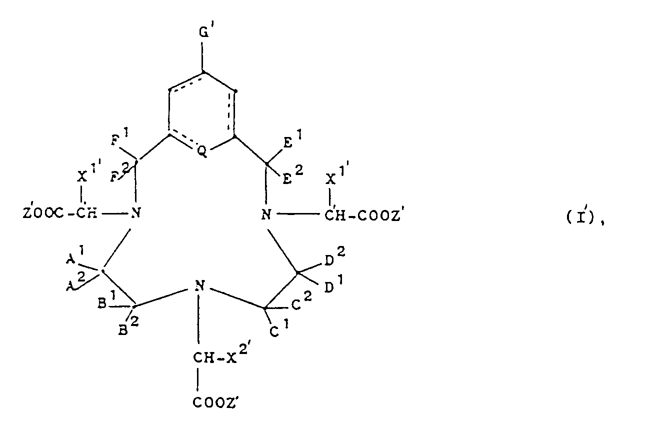 worin
G' X^{1'} und X^{2'} jeweils für G, X¹ und X², bei denen die darin enthaltenen Hydroxy- und funktionellen Gruppen in geschützter Form bzw. als Vorstufe vorliegen, und
Z' für ein Wasserstoffatom oder eine Säureschutzgruppe
stehen,
die Schutzgruppen abspaltet, gegebenenfalls die gewünschte funktionelle Gruppe generiert, gewünschtenfalls die so erhaltenen Komplexbildner der allgemeinen Formel I mit Z in der Bedeutung von Wasserstoff in an sich bekannter Weise mit mindestens einem Metalloxid oder Metallsalz eines Elements der Ordnungszahlen 21-29, 31, 32, 37-39, 42-44, 49 oder 57-83 umsetzt, gewünschtenfalls die funktionellen Gruppen an ein Bio- oder Makromolekül bindet - wobei der Komplexierung vor oder nach der Abspaltung der Schutzgruppen für die Hydroxy- und funktionellen Gruppen bzw. Generierung der funktionellen Gruppen und Bindung an ein Makro-oder Biomolekül erfolgen kann - und anschließend, falls gewünscht, noch vorhandene acide Wasserstoffatome durch Kationen anorganischer und/oder organischer Basen, Aminosäuren oder Aminosäureamide substituiert bzw. die entsprechenden Säuregruppen ganz oder teilweise in Ester oder Amide überführt.

14. Pharmazeutische Mittel enthaltend mindestens eine Verbindung nach Anspruch 1, gegebenenfalls mit den in der Galenik üblichen Zusätzen.

15. Pharmazeutische Mittel enthaltend mindestens eine Verbindung nach Anspruch 1 in Form von Liposomen.

16. Verfahren zur Herstellung der pharmazeutischen Mittel gemäß Anspruch 14, dadurch gekennzeichnet, daß man die in Wasser oder physiologischer Salzlösung gelöste oder suspendierte Komplexverbindung, gegebenenfalls mit den in der Galenik üblichen Zusätzen, in eine für die enterale oder parenterale Applikation geeignete Form bringt.

## Claims

1. Macrocyclic compounds of the general formula I 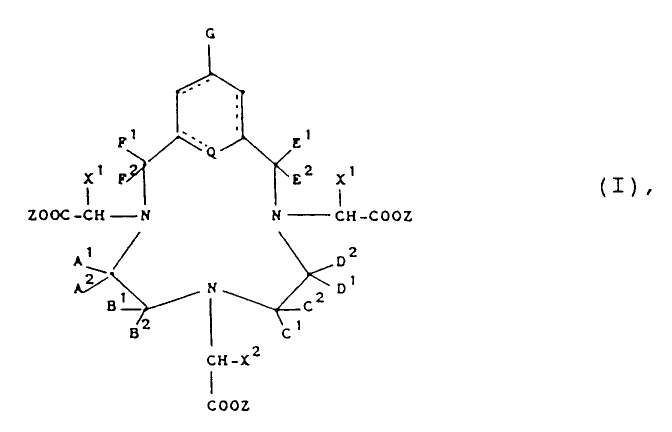 wherein
... represents a single or double bond,
Q represents a nitrogen atom or the radical NH,
X¹ represents a hydrogen atom, a group -(CH₂)ₙ-R¹ or 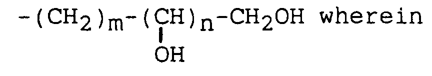 wherein
n represents a number from 1 to 5,
m represents a number from 0 to 2, and
R¹ represents a hydrogen atom or a hydroxy group,
X² represents X¹ or a group -(CH₂)ₙ-(O)₁-(CH₂)ₖ-(C₆H₄)_{q}-R² wherein
k represents a number from 0 to 4,
each of l and q represents the number 0 or 1, and
R² represents a hydrogen atom, a C₁-C₄alkoxy group, a functional group or a bio- or macro-molecule bound by way of that functional group,
A¹, A², B¹, B², C¹, C², D¹, D², E¹, E², F¹ and F² each independently of the other represent X²,
G represents R² or a second macrocycle, bound by way of K, of the general formula II 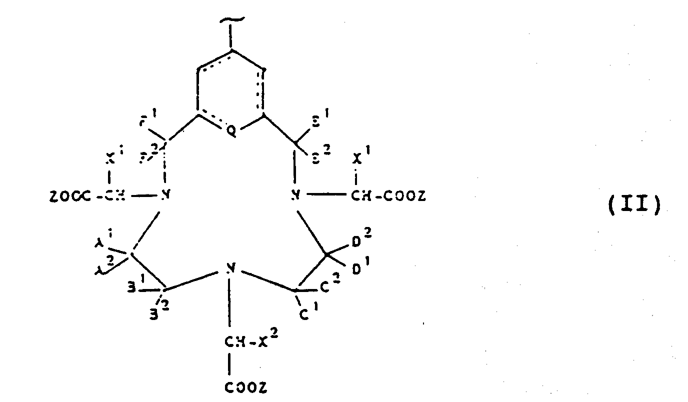 wherein K represents a direct bond, a bis(carbonylamino) group (-NH-CO-CO-NH-) or a C₁-C₁₄alkylene group which optionally carries at its ends carbonyl (>CO) or carbonylamino (-NH-CO-) groups or oxygen atoms and which optionally contains one or more oxygen atom(s), hydroxymethylene (-CH(OH)-) group(s), CH(X²)COOZ-, acyl-or hydroxyacyl-substituted imino groups or one or two C-C double bonds and/or C-C triple bonds,
Z represents a hydrogen atom and/or a metal ion equivalent of an element of atomic number 21-29, 31, 32, 37-39, 42-44, 49 or 57-83,
with the proviso that the 12 ring substituents A¹ to F² represent at least 8 hydrogen atoms, that X¹ and X² simultaneously represent hydrogen atoms only when at least one of the ring substituents A¹ to F² does not represent a hydrogen atom, at least one of the ring substituents A¹ to D² does not represent (CH₂)₁₋₆-H and at least one of the ring substituents E¹ to F² does not represent (CH₂)₁₋₅-H, and that the macrocycle of the general formula I contains not more than one bio- or macro-molecule and that, if desired, remaining CO₂H groups are present in the form of an ester or amide, and also salts thereof with inorganic and/or organic bases, amino acids or amino acid amides.

2. Macrocyclic compounds of the general formula III 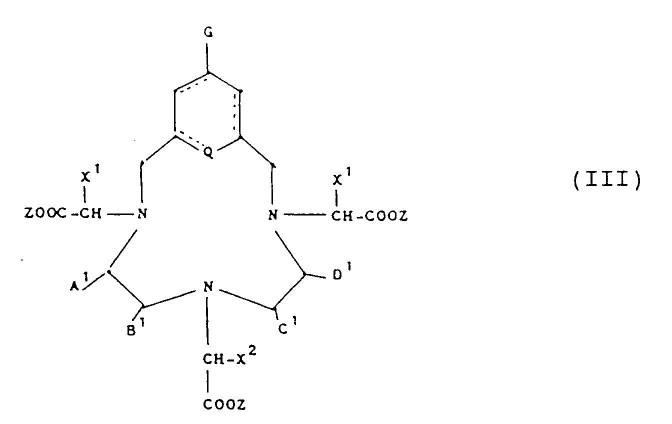 wherein
... represents a single or double bond,
Q represents a nitrogen atom or the radical NH,
X¹ represents a hydrogen atom, a group -(CH₂)ₙ-R¹ or 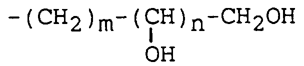 wherein
n represents a number from 1 to 5,
m represents a number from 0 to 2, and
R¹ represents a hydrogen atom or a hydroxy group,
X² represents X¹ or a group -(CH₂)ₙ-(O)₁-(CH₂)ₖ-(C₆H₄)_{q}-R² wherein
k represents a number from 0 to 4,
each of l and q represents the number 0 or 1, and
R² represents a hydrogen atom, a C₁-C₄alkoxy group, a functional group or a bio- or macro-molecule bound by way of that functional group,
A¹, B¹, C¹ and D¹ each independently of the other represent X²,
G represents R² or a second macrocycle, bound by way of K, of the general formula IV 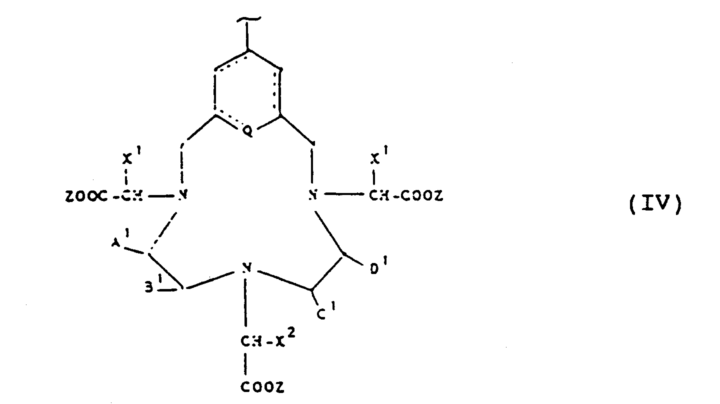 wherein K represents a direct bond, a bis(carbonylamino) group (-NH-CO-CO-NH-) or a C₁-C₁₄alkylene group which optionally carries at its ends carbonyl (>CO) or carbonylamino (-NH-CO-) groups or oxygen atoms and which optionally contains one or more oxygen atom(s), hydroxymethylene (-CH(OH)-) group(s), CH(X²)COOZ-, acyl- or hydroxyacyl-substituted imino groups or one or two C-C double bonds and/or C-C triple bonds,
Z represents a hydrogen atom and/or a metal ion equivalent of an element of atomic number 21-29, 31, 32, 37-39, 42-44, 49 or 57-83,
with the proviso that X¹ and X² simultaneously represent hydrogen atoms only when at least one of the 4 ring substituents A¹, B¹, C¹ and D¹ does not represent a hydrogen atom or (CH₂)₁₋₆-H and that, if desired, remaining CO₂H groups are present in the form of an ester or amide, and also salts thereof with inorganic and/or organic bases, amino acids or amino acid amides.

3. Compounds according to claim 1, characterised in that Z represents hydrogen atoms.

4. Compounds according to claim 1, characterised in that at least 2 of the substituents Z are metal ion equivalents of at least one element of atomic numbers 21-29, 42, 44 or 57-83 or of at least one radionuclide of an element of atomic number 27, 29, 31, 32, 37-39, 43, 49, 62, 64, 70 or 77.

5. Compounds according to claim 1, characterised in that K represents a group 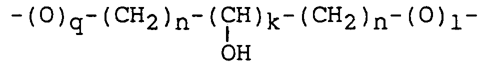 or a direct bond.

6. Compounds according to claim 1, characterised in that the functional group representing R² is NCS, NO₂, OH, NHNH₂, NH₂, NHCOCH₂Br, NHCOCH₂Cl, CO₂H or CON₃.

7. Compounds according to claim 1, characterised in that the bio- or macro-molecule optionally contained in R² is an antibody or antibody fragment.

8. Compounds according to claim 1, characterised in that the bio- or macro-molecule optionally contained in R² is a protein, such as albumin, globulin or lectin.

9. Compounds according to claim 1, characterised in that the bio- or macro-molecule optionally contained in R² is a polysaccharide, such as a starch, dextran or dextrin.

10. Compounds according to claim 1, characterised in that X¹ represents CH₂OH, CH₂CH₂OH or CHOHCH₂OH.

11. Compounds according to claim 2, characterised in that X², A¹, B¹, C¹ and/or D¹ represent(s) CH₂OH, CH₂CH₂OH, CH₂OCH₂C₆H₅, CHOHCH₂OH, CH₂C₆H₄OCH₃, CH₂C₆H₅, CH₂C₆H₄O(CH₂)₃COOH or CH₂C₆H₄NCS.

12. Use of at least one physiologically tolerable compound according to claim 4 for the preparation of agents for NMR-, X-ray- or radio-diagnostics or for radioimmuno- or radio-therapy.

13. Process for the preparation of macrocyclic compounds of the general formula I 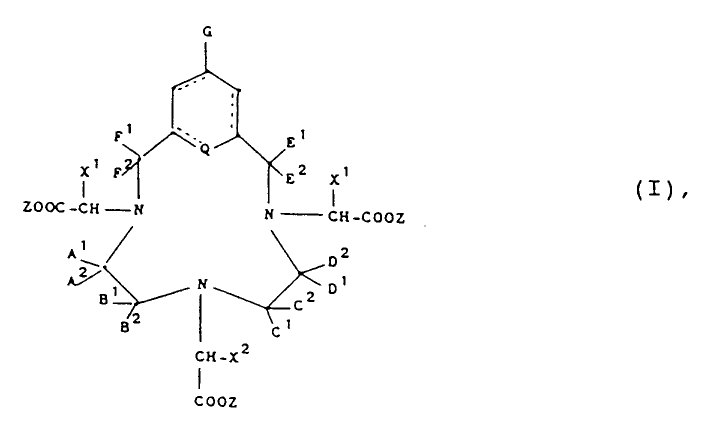 wherein
... represents a single or double bond,
Q represents a nitrogen atom or the radical NH,
X¹ represents a hydrogen atom, a group -(CH₂)ₙ-R¹ or 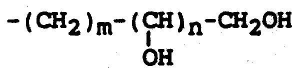 wherein
n represents a number from 1 to 5,
m represents a number from 0 to 2, and
R¹ represents a hydrogen atom or a hydroxy group,
X² represents X¹ or a group -(CH₂)ₙ-(O)₁-(CH₂)ₖ-(C₆H₄)_{q}-R² wherein
k represents a number from 0 to 4,
each of l and q represents the number 0 or 1, and
R² represents a hydrogen atom, a C₁-C₄alkoxy group, a functional group or a bio- or macro-molecule bound by way of that functional group,
A¹, A², B¹, B², C¹, C², D¹, D², E¹, E², F¹ and F² each independently of the other represent X²,
G represents R² or a second macrocycle, bound by way of K, of the general formula II 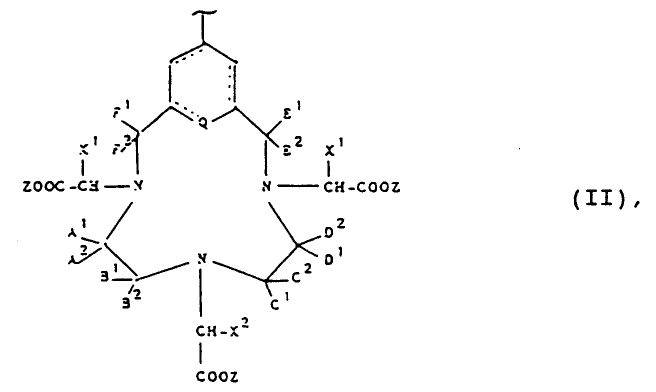 wherein K represents a direct bond, a bis(carbonylamino) group (-NH-CO-CO-NH-) or a C₁-C₁₄alkylene group which optionally carries at its ends carbonyl (>CO) or carbonylamino (-NH-CO-) groups or oxygen atoms and which optionally contains one or more oxygen atom(s), hydroxymethylene (-CH(OH)-) group(s), CH(X²)COOZ-, acyl- or hydroxyacyl-substituted imino groups or one or two C-C double bonds and/or C-C triple bonds,
Z represents a hydrogen atom and/or a metal ion equivalent of an element of atomic number 21-29, 31, 32, 37-39, 42-44, 49 or 57-83,
with the proviso that the 12 ring substituents A¹ to F² represent at least 8 hydrogen atoms, that X¹ and X² simultaneously represent hydrogen atoms only when at least one of the ring substituents A¹ to F² does not represent a hydrogen atom, at least one of the ring substituents A¹ to D² does not represent (CH₂)₁₋₆-H and at least one of the ring substituents E¹ to F² does not represent (CH₂)₁₋₅-H, and that the macrocycle of the general formula I contains not more than one bio- or macro-molecule and that, if desired, remaining CO₂H groups are present in the form of an ester or amide, and also salts thereof with inorganic and/or organic bases, amino acids or amino acid amides,
characterised in that, in a manner known per se, in compounds of the general formula I' 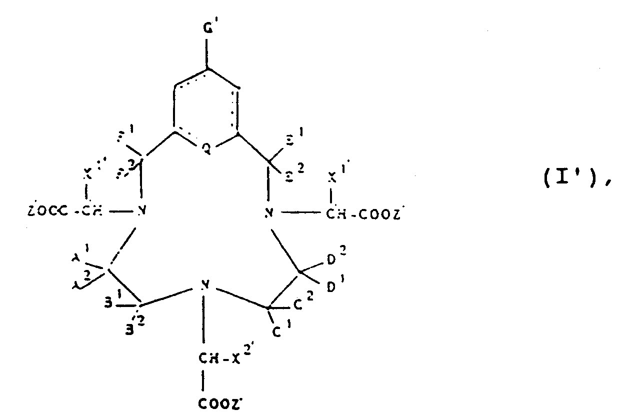 wherein
G', X^{1'} and X^{2'} represent G, X¹ and X², respectively, wherein the hydroxy groups and functional groups are present in protected form or in the form of precursor groups, and
Z' represents a hydrogen atom or an acid-protecting group,
the protecting groups are removed, optionally the desired functional group is generated, if desired the resulting complex formers of the general formula I wherein Z represents hydrogen are reacted in a manner known per se with at least one metal oxide or metal salt of an element of atomic number 21-29, 31, 32, 37-39, 42-44, 49 or 57-83, if desired the functional groups are bound to a bio- or macro-molecule, it being possible for the complexing to be effected either before or after removal of the protecting groups for the hydroxy groups and functional groups and either before or after the generation of the functional groups and binding to a macro- or bio-molecule, and then, if desired, any acidic hydrogen atoms that remain are replaced by cations of inorganic and/or organic bases, amino acids or amino acid amides and/or all or some of the corresponding acid groups are converted into esters or amides.

14. Pharmaceutical agents comprising at least one compound according to claim 1, optionally with additives customary in galenic pharmacy.

15. Pharmaceutical agents comprising at least one compound according to claim 1 in the form of liposomes.

16. Process for the preparation of the pharmaceutical agents according to claim 14, characterised in that the complex compound, which is dissolved or suspended in water or physiological saline, optionally with additives customary in galenic pharmacy, is rendered into a form that is suitable for enteral or parenteral administration.

## Revendications

1. Composés macrocycliques de formule générale I : 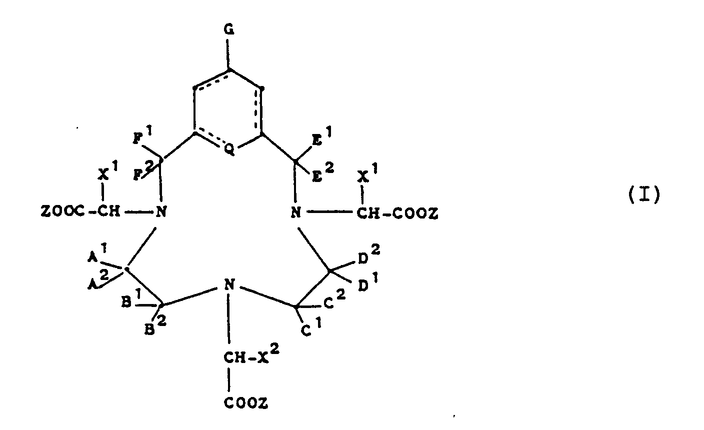 où
... représente une simple ou double liaison,
Q représente un atome d'azote ou le radical NH,
X¹ représente un atome d'hydrogène, un groupe -(CH₂)ₙ-R¹- ou 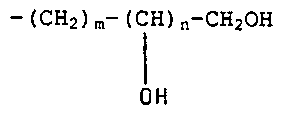 avec
n ayant la signification des nombres de 1 à 5,
m ayant la signification des nombres de 0 à 2, et
R¹ ayant la signification d'un atome d'hydrogène ou d'un groupe hydroxy,
X² représente X¹ ou un groupe -(CH₂)ₙ-(O)₁-(CH₂)ₖ-(C₆H₄)_{q}-R² avec
k ayant la signification des nombres de 0 à 4,
l et q ayant la signification des nombres de 0 ou 1, et
R² ayant la signification d'un atome d'hydrogène, d'un groupe alcoxy en C₁-C₄, d'un groupe fonctionnel ou d'une bio- ou macromolécule liée par l'intermédiaire de ce groupe fonctionnel,
A¹, A², B¹, B², C¹, C², D¹, D², E¹, E², F¹ et F², indépendamment les uns des autres, représentent chacun X²,
G représente R² ou un deuxième macrocycle lié par l'intermédiaire de K de formule générale II : 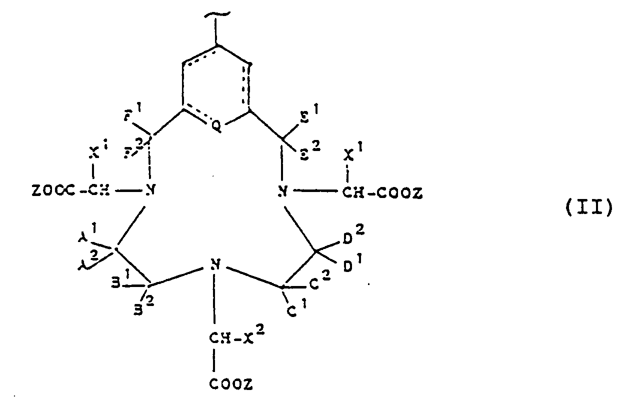 avec K ayant la signification d'une liaison directe, d'un groupe bis-(carbonylamino) (-NH-CO-CO-NH-) ou d'un groupe alkylène en C₁-C₁₄, qui porte éventuellement à ses extrémités des groupes carbonyle (>CO) ou carbonylamino (-NH-CO-) ou des atomes d'oxygène et contient éventuellement un ou plusieurs atomes d'oxygène, des groupes hydroxyméthylène (-CH-OH-), des groupes imino substitués par CH(X²)COOZ, acyle ou hydroxyacyle ou contient une à deux doubles liaisons C-C et/ou triples liaisons C-C,
Z représente un atome d'hydrogène et/ou un équivalent d'ion métallique d'un élément ayant le nombre atomique 21-29, 31, 32, 37-39, 42-44, 49 ou 57-83,
à la condition que les 12 substituants sur le cycle A¹ à F² représentent au moins 8 atomes d'hydrogène, que X¹ et X² ne soient simultanément des atomes d'hydrogène, que si au moins un des substituants sur le cycle A¹ à F² ne représente pas un atome d'hydrogène A¹ à D² ne représentent pas (CH₂)₁₋₆-H et E¹ à F² ne représentent pas (CH₂)₁₋₅-H et que le macrocycle de formule générale I ne contienne pas plus d'une bio- ou macromolécule et, si on le désire, le radical des groupes CO₂H soit présent sous forme d'ester ou d'amide, ainsi que leurs sels avec des bases organiques et/ou minérales, des acides aminés ou des amides d'acides aminés.

2. Composés macrocycliques de formule générale III : 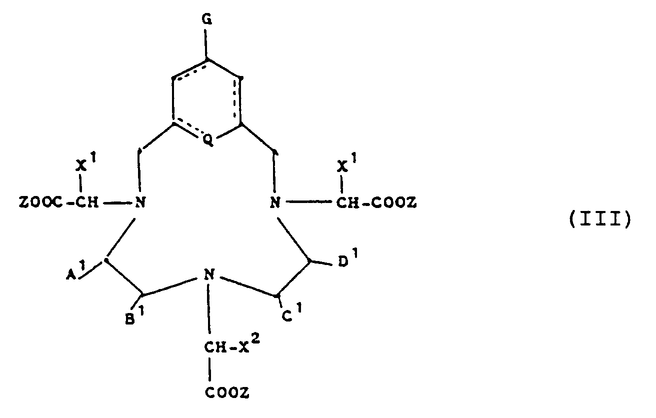 où
... représente une simple ou double liaison,
Q représente un atome d'azote ou le radical NH,
X¹ représente un atome d'hydrogène, un groupe -(CH₂)ₙ-R¹- ou 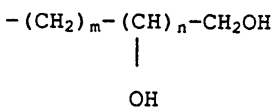 avec
n ayant la signification des chiffres de 1 à 5,
m ayant la signification des chiffres de 0 à 2, et
R¹ ayant la signification d'un atome d'hydrogène ou d'un groupe hydroxy,
X² représente X¹ ou un groupe -(CH₂)ₙ-(O)₁-(CH₂)ₖ-(C₆H₄)_{q}-R² avec
k ayant la signification des chiffres de 0 à 4,
l et q ayant la signification des chiffres de 0 ou 1, et
R² ayant la signification d'un atome d'hydrogène, d'un groupe alcoxy en C₁-C₄, d'un groupe fonctionnel ou d'une bio- ou macromolécule liée par l'intermédiaire de ce groupe fonctionnel,
A¹, B¹, C¹ et D¹, indépendamment les uns des autres, représentent chacun X²,
G représente R² ou un deuxième macrocycle lié par l'intermédiaire de K de formule générale IV : 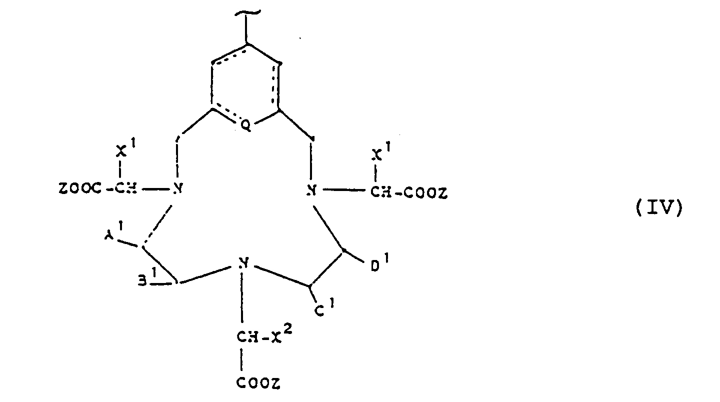 avec K ayant la signification d'une liaison directe, d'un groupe bis-(carbonylamino) (-NH-CO-CO-NH-) ou d'un groupe alkylène en C₁-C₁₄, qui porte éventuellement à ses extrémités des groupes carbonyle (>CO) ou carbonylamino (-NH-CO-) ou des atomes d'oxygène et contient éventuellement un ou plusieurs atomes d'oxygène, des groupes hydroxyméthylène (-CH-OH-), des groupes imino substitués par CH(X²)COOZ, acyle ou hydroxyacyle ou contient une à deux doubles liaisons C-C et/ou triples liaisons C-C,
Z représente un atome d'hydrogène et/ou un équivalent d'ion métallique d'un élément ayant le nombre atomique 21-29, 31, 32, 37-39, 42-44, 49 ou 57-83,
à la condition que X¹ ou (CH₂)₁₋₆-H et X² ne soient simultanément des atomes d'hydrogène que si au moins un des quatre substituants sur le cycle A¹, B¹, C¹ et D¹ ne représente pas un atome d'hydrogène ou (CH₂)₁₋₆-H et que, si on le désire, le radical des groupes CO₂H soit présent sous forme d'ester ou d'amide, ainsi que leurs sels avec des bases organiques et/ou minérales, des acides aminés ou des amides d'acides aminés.

3. Composés selon la revendication 1, caractérisés en ce que Z représente des atomes d'hydrogène.

4. Composés selon la revendication 1, caractérisés en ce qu'au moins deux des substituants Z sont des équivalents d'ions métalliques d'au moins un des éléments ayant le nombre atomique 21 à 29, 42, 44 ou de 57 à 83 ou au moins l'un des radionuclides d'un des éléments ayant le nombre atomique 27, 29, 31, 32, 37 à 39, 43, 49, 62, 64, 70 ou 77.

5. Composés selon la revendication 1, caractérisésen ce que K représente un groupe 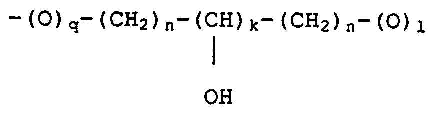 ou une

6. Composés selon la revendication 1, caractérisés en ce que le groupe fonctionnel correspondant à R² représente NCS, NO₂, OH, NHNH₂, NH₂, NHCOCH₂Br, NHCOCH₂Cl, CO₂H, CON₃.

7. Composés selon la revendication 1, caractérisés en ce que la bio- ou macromolécule contenue éventuellement dans R² est un anticorps ou un fragment d'anticorps.

8. Composés selon la revendication 1, caractérisés en ce que la bio- ou macromolécule contenue éventuellement dans R² est une protéine, comme l'albumine, la globuline ou la lectine.

9. Composés selon la revendication 1, caractérisés en ce que la bio- ou macromolécule contenue éventuellement dans R² est un polysaccharide comme l'amidon, le dextran ou la dextrine.

10. Composés selon la revendication 1, caractérisés en ce que X¹ représente CH₂OH, CH₂CH₂OH, CHOHCH₂OH.

11. Composés selon la revendication 2, caractérisés en ce que X², A¹, B¹, C¹ et/ou D¹ représentent CH₂OH, CH₂CH₂OH, CH₂OCH₂C₆H₅, CHOHCH₂OH, CH₂C₆H₄OCH₃, CH₂C₆H₅, CH₂C₆H₄O(CH₂)₃COOH, CH₂C₆H₄NCS.

12. Utilisation d'au moins un composé physiologiquement compatible selon la revendication 4 pour la préparation d'agents pour le diagnostic par la RMN, aux rayons X et radiodiagnostic, pour la thérapie radioimmunologique ou par irradiation.

13. Procédé pour la préparation de composés macrocycliques de formule générale I : 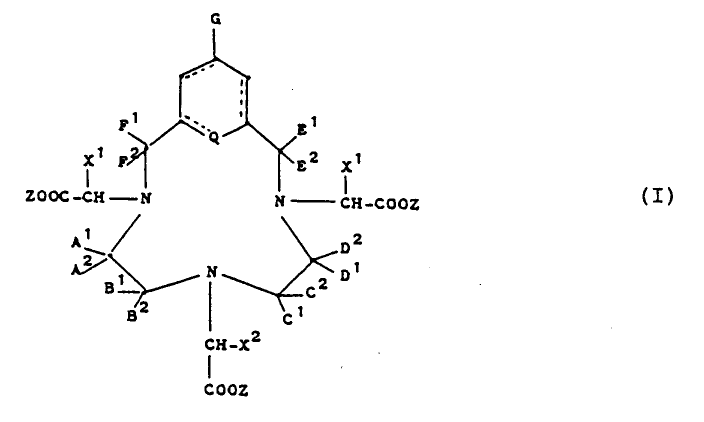 où
... représente une simple ou double liaison,
Q représente un atome d'azote ou le radical NH,
X¹ représente un atome d'hydrogène, un groupe -(CH₂)ₙ-R¹- ou 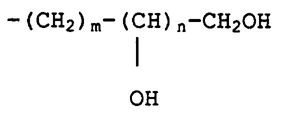 avec
n ayant la signification des nombres de 1 à 5,
m ayant la signification des nombres de 0 à 2, et
R¹ ayant la signification d'un atome d'hydrogène ou d'un groupe hydroxy,
X² représente X¹ ou un groupe -(CH₂)ₙ-(O)₁-(CH₂)ₖ-(C₆H₄)_{q}-R² avec
k ayant la signification des nombre de 0 à 4,
l et q ayant la signification des nombres de 0 ou 1, et
R² ayant la signification d'un atome d'hydrogène, d'un groupe alcoxy en C₁-C₄, d'un groupe fonctionnel ou d'une bio- ou macromolécule liée par l'intermédiaire de ce groupe fonctionnel,
A¹, A², B¹, B², C¹, C², D¹, D², E¹, E², F¹ et F², indépendamment les uns des autres, représentent chacun X²,
G représente R² ou un deuxième macrocycle lié par l'intermédiaire de K de formule générale II : 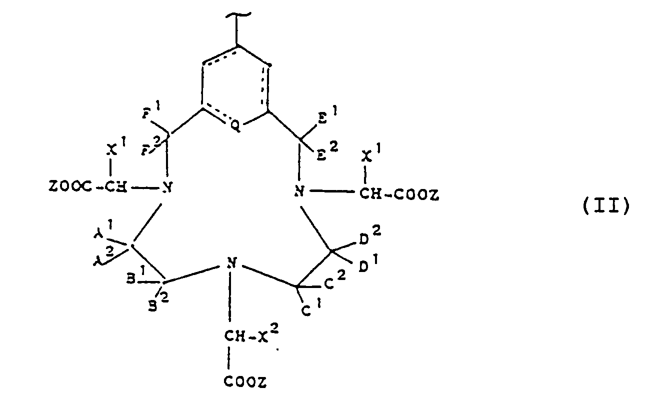 avec K ayant la signification d'une liaison directe, d'un groupe bis-(carbonylamino) (-NH-CO-CO-NH-) ou d'un groupe alkylène en C₁-C₁₄, qui porte éventuellement à ses extrémités des groupes carbonyle (>CO) ou carbonylamino (-NH-CO-) ou des atomes d'oxygène et contient éventuellement un ou plusieurs atomes d'oxygène, des groupes hydroxyméthylène (-CH-OH-), des groupes imino substitués par CH(X²)COOZ, acyle ou hydroxyacyle ou une à deux doubles liaisons C-C et/ou triples liaisons C-C,
Z représente un atome d'hydrogène et/ou un équivalent d'ion métallique d'un élément ayant le nombre atomique 21-29, 31, 32, 37-39, 42-44, 49 ou 57-83,
à la condition que les 12 substituants sur le cycle A¹ à F² représentent au moins 8 atomes d'hydrogène, que X¹ et X² ne soient simultanément des atomes d'hydrogène que si au moins un des substituants sur le cycle A¹ à F² ne représente pas un atome d'hydrogène A¹ à D² ne représentent pas (CH₂)₁₋₆-H et E¹ à F² ne représentent pas (CH₂)₁₋₅-H et que le macrocycle de formule générale I ne contient pas plus d'une bio- ou macromolécule et, si on le désire, le radical des groupes CO₂H soit présent sous forme d'ester ou d'amide, ainsi que leurs sels avec des bases organiques et/ou minérales, des acides aminés ou des amides d'acides aminés caractérisé en ce que l'on sépare de façon connue en soi des composés de formule générale I' : 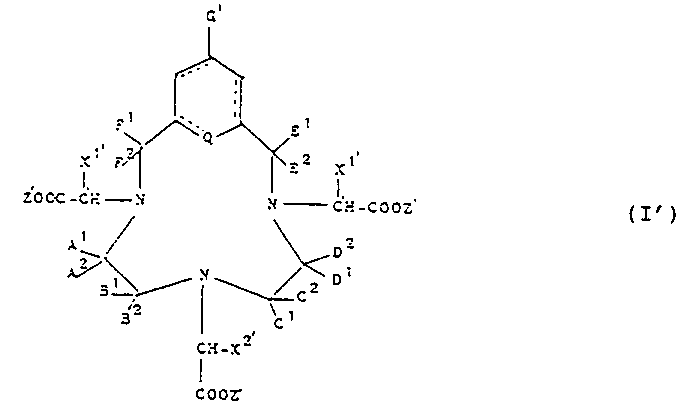 dans laquelle
G', X^{1'} et X^{2'} chacun représentent G, X¹ et X² dans lesquels les groupes fonctionnels et hydroxy contenus sont présents sous forme protégée, respectivement comme précurseurs, et
Z' représente un atome d'hydrogène ou un groupe protecteur d'acides,
les groupes protecteurs, éventuellement on génère le groupe fonctionnel désiré, si on le souhaite on fait réagir les agents complexants ainsi obtenus de formule générale I avec Z ayant la signification d'hydrogène de façon connue en soi avec au moins un oxyde métallique ou sel métallique d'un élément ayant le nombre atomique 21 à 29, 31, 32, 37 à 39, 42 à 44, 49 ou 57 à 83, si on le souhait on lie les groupes fonctionnels à une bio- ou macromolécule - la complexation pouvant être effectuée avant ou après la séparation des groupes protecteurs pour les groupes fonctionnels et hydroxyle, respectivement la génération des groupes fonctionnels et la liaison à une bio- ou macromolécule - et finalement, si on le désire, on peut substituer les atomes d'hydrogène acides encore présents par des cations de bases minérales et/ou organiques, des acides aminés ou des amides d'acides aminés, respectivement on peut transformer les groupes acides correspondants entièrement ou partiellement en esters ou amides.

14. Agents pharmaceutiques contenant au moins un composé selon la revendication 1, éventuellement avec des adjuvants usuels en galénique.

15. Agents pharmaceutiques contenant au moins un composé selon la revendication 1 sous forme de liposomes.

16. Procédé pour la préparation d'agents pharmaceutiques selon la revendication 14, caractérisé en ce que l'on met en forme appropriée le composé complexe suspendu ou dissous dans l'eau ou dans une solution physiologique de sels, éventuellement avec des adjuvants usuels en galénique, en une forme apporpriée pour application entérale ou parentérale.
